# EUROPÄISCHE PATENTSCHRIFT

(11) **EP 0 938 514 B1**
(45) Veröffentlichungstag und Bekanntmachung des Hinweises auf die Patenterteilung: **18.12.2002**
(21) Anmeldenummer: 97950074.1
(22) Anmeldetag: 03.11.1997
(51) Int. Cl.: C08G 61/00, C09K 11/06

(54) **GEORDNETE POLY(ARYLENVINYLEN)-TERPOLYMERE, VERFAHREN ZU IHRER HERSTELLUNG UND IHRE VERWENDUNG ALS ELEKTROLUMINESZENZMATERIALIEN**
ORDERED POLY(ARYLENE VINYLENE) TERPOLYMERS, METHOD FOR THE PRODUCTION AND THE USE THEREOF AS ELECTROLUMINESCENT MATERIALS
TERPOLYMERES DE POLY(ARYLENEVINYLENE) ORDONNES, LEUR PROCEDE DE PREPARATION ET LEUR UTILISATION COMME MATERIAUX ELECTROLUMINESCENTS

(30) Priorität: 13.11.1996 DE 19646877
(43) Veröffentlichungstag der Anmeldung: 01.09.1999
(73) Patentinhaber: Covion Organic Semiconductors GmbH, 65926 Frankfurt (DE)
(72) Erfinder: KREUDER, Willi, D-55126 Mainz (DE); HÖRHOLD, Hans-Heinrich, D-07747 Jena (DE); ROST, Henning, D-07743 Jena (DE); HARTMANN, Annett, D-07747 Jena (DE)
(74) Vertreter: Luderschmidt, Schüler & Partner GbR
(86) Internationale Anmeldenummer: EP9706051
(87) Internationale Veröffentlichungsnummer: WO98021262

(56) Entgegenhaltungen:
- EP-A- 0 637 621
- EP-A- 0 672 741

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

In WO 90/13148 und EP-A 0 443 861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile, wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Gemäß WO-90/13148 wird als polymeres Material für die lichtemitierende Schicht Poly(p-phenylenvinylen) (PPV) verwendet, und es wird vorgeschlagen, die Phenylgruppe in einem solchen Material zu substituieren oder durch andere, carbocyclische oder heterocyclische aromatische Ringsysteme zu ersetzen.

Poly(p-phenylenvinylen)-Derivate zur Verwendung als Elektrolumineszenzmaterialien sind beispielsweise auch aus WO/A 96/10617 und PCT/EP 96/01066 bekannt.

Obwohl mit solchen Materialien gute Ergebnisse erzielt wurden, ist das Eigenschaftsprofil dieser Polymere noch durchaus verbesserungsfähig.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen nach wie vor an den unterschiedlichsten Elektrolumineszenzmaterialien für solche Vorrichtungen interessiert.

Dies liegt unter anderem auch daran, daß erst das Zusammenwirken des Elektrolumineszenzmaterials mit den weiteren Bauteilen der Vorrichtungen Rückschlüsse auf die Eignung auch des Elektrolumineszenzmaterials zuläßt.

Aufgabe der vorliegenden Erfindung war es daher, neue Elektrolumineszenzmaterialien bereitzustellen, die bei Verwendung in Beleuchtungs- oder Anzeigevorrichtungen geeignet sind, das Eigenschaftsprofil dieser Vorrichtungen zu verbessern.

Es wurde nun überraschend gefunden, daß sich bestimmte geordnete Poly-(arylenvinylen)-Terpolymere in besonderer Weise als Elektrolumineszenzmaterialien eignen.

Ein Gegenstand der Erfindung ist daher ein Terpolymer, enthaltend Wiederholeinheiten der Formel (I), 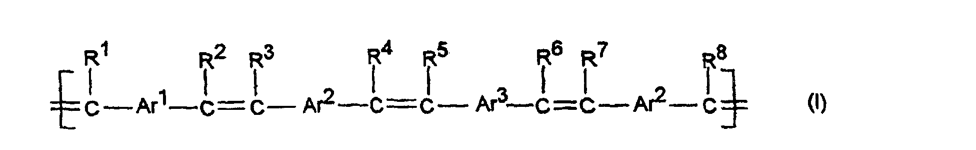 wobei die Symbole folgende Bedeutungen haben:
- Ar¹, Ar², Ar³: sind gleich oder verschieden ein- oder mehrkemige, gegebenenfalls substituierte und gegebenenfalls über eine oder mehrere, vorzugsweise eine, Brücke verknüpfte, oder kondensierte Aryl- oder Heteroarylgruppen, vorzugsweise mit 2 bis 100, besonders bevorzugt 2 bis 50, ganz besonders bevorzugt 2 bis 20 Kohlenstoffatomen;
- R¹, R², R³, R⁴, R⁵, R⁶, R⁷, R⁸: sind gleich oder verschieden H oder ein Kohlenwasserstoffrest mit 1 bis 22 Kohlenstoffatomen, der gegebenenfalls substituiert sein kann, vorzugsweise mit F, und auch Heteroatome, vorzugsweise O, enthalten kann;
mit der Maßgabe, daß =CR¹-Ar¹-CR²=, =CR³-Ar²-CR⁴= und CR⁵-Ar³-CR⁶= jeweils verschieden voneinander sind.

Verschieden bedeutet im Sinne der Erfindung, daß es sich zumindest um Regio- oder Stereoisomere handelt oder die Reste Ar¹, Ar², Ar³ zumindest unterschiedlich substituiert sind.

Terpolymere im Sinne der Erfindung sind ternäre Copolymere.

Die erfindungsgemäßen Polymere weisen im allgemeinen 2 bis 1000, vorzugsweise 3 bis 500, besonders bevorzugt 4 bis 300, Wiederholeinheiten, vorzugsweise der Formel (I), auf.

Bevorzugt sind Polymere, bestehend aus Wiederholeinheiten der Formel (I).

Weiterhin bevorzugt sind Polymere, enthaltend Wiederholeinheiten der Formel (I), bei denen R⁷ und R⁸ paarweise gleich R³ bzw. R⁴ sind.

Ebenso bevorzugt sind Polymere, enthaltend Wiederholeinheiten der Formel (I), bei denen die Symbole folgende Bedeutungen haben:
- Ar¹, Ar², Ar³: sind gleich oder verschieden 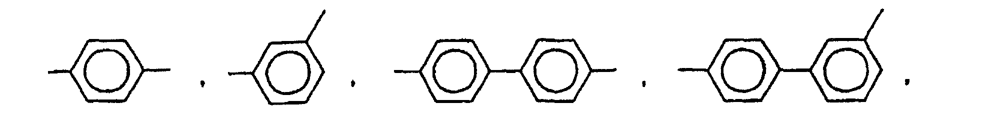 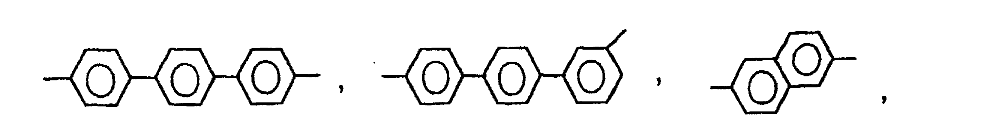 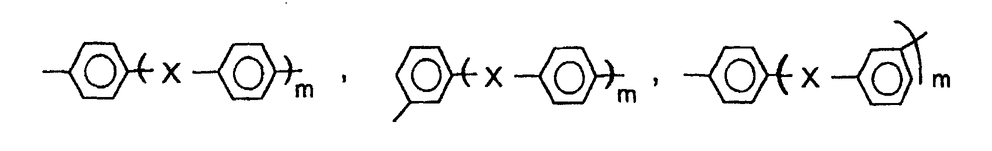 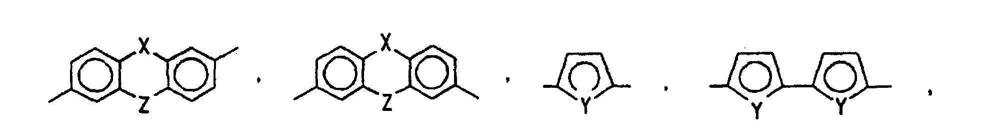 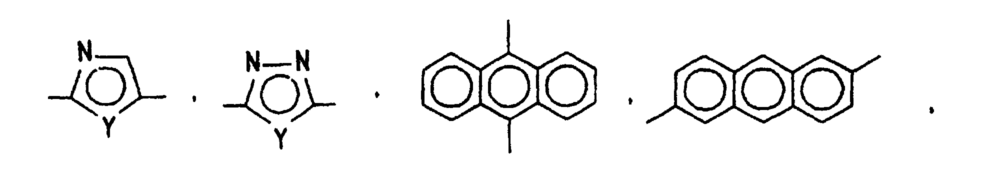 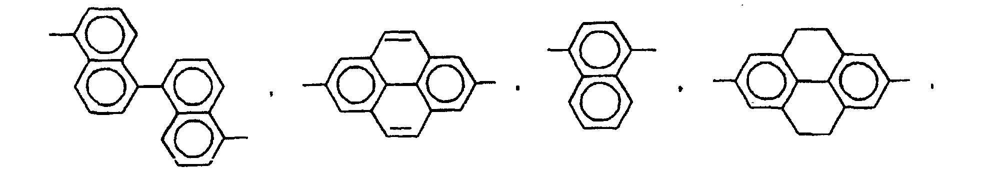 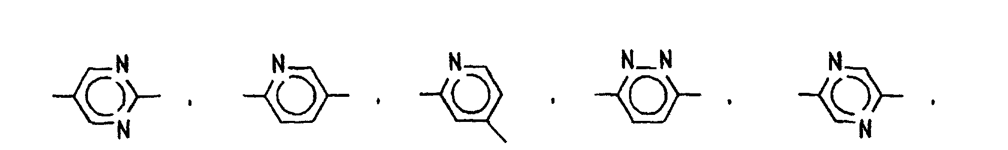 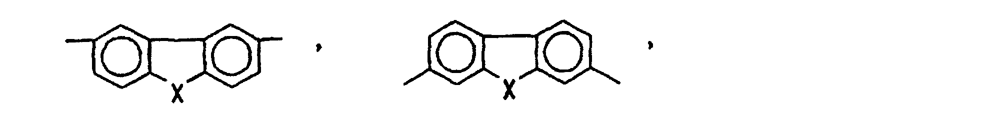
- Ar¹, Ar², Ar³: können dabei gleich oder verschieden mit einem oder mehreren Resten R⁹-R¹⁴ substituiert sein,
wobei die obige Maßgabe gilt;
- m: ist 1 bis 20, vorzugsweise 1, 2 oder 3, besonders bevorzugt 1;
- X, Z: sind gleich oder verschieden eine Einfachbindung, -O-, -S-, -SO-, -SO₂-, -CO-, -CR⁹R¹⁰-, -CR¹¹=CR¹²-, -CHR¹³, -CHR¹⁴-, -NR¹⁵-;
- Y: ist -O-, -S-, oder -NR¹⁵-;
- R⁹-R¹⁴: sind gleich oder verschieden H, -CF₃, -Ph, -O-Ph, -S-Ph, -SO-Ph, -SO₂-Ph, F, Cl, Br, I, -CN oder eine Alkylgruppe mit 1 bis 22, vorzugsweise 1 bis 12, Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können;
- R¹⁵: hat, gleich oder verschieden von R¹, die gleichen Bedeutungen wie R¹;
- n: ist 0, 1 oder 2, vorzugsweise 0 oder 1, besonders bevorzugt 0.

Besonders bevorzugt sind Polymere, enthaltend Wiederholeinheiten der Formel (I), bei denen die Symbole und Indizes folgende Bedeutungen haben:
- Ar¹, Ar², Ar³: sind gleich oder verschieden 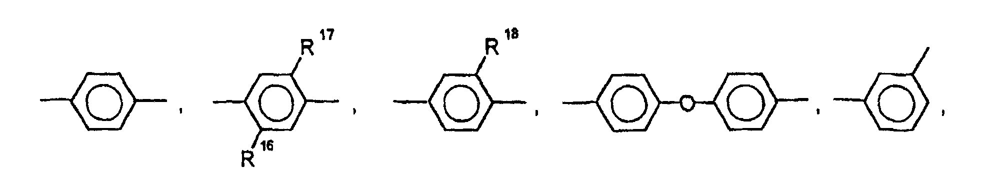 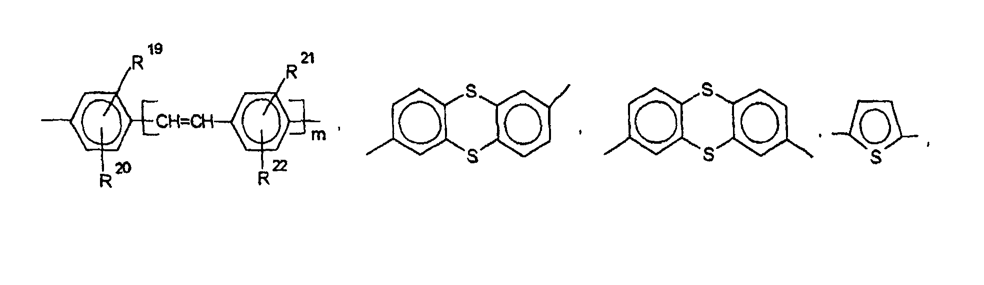 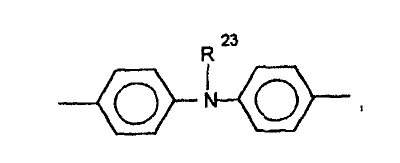 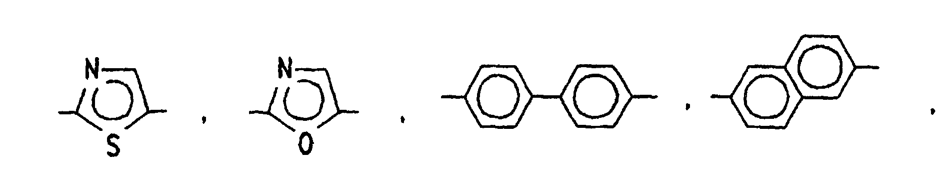
wobei die obige Maßgabe gilt;
- m: ist 1 bis 20, vorzugsweise 1, 2 oder 3, besonders bevorzugt 1;
- R¹⁶-R²³: sind gleich oder verschieden F, Cl, C₆-C₁₀-Aryl, eine geradkettige oder verzweigte Alkyl- oder Alkoxygruppe mit 1 bis 22, vorzugsweise 1 bis 12, Kohlenstoffatomen, R¹⁹⁻²² können auch H sein.

Ganz besonders bevorzugt sind
- Ar¹, Ar² und Ar³: gleich oder verschieden 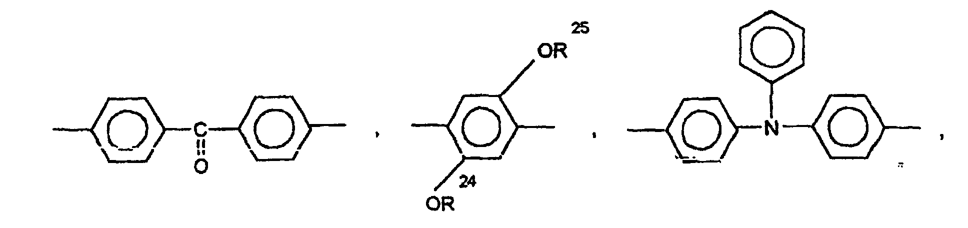 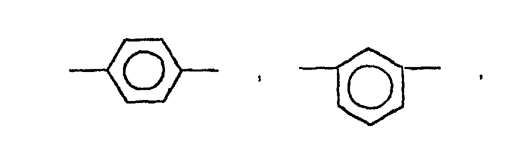
wobei die obige Maßgabe gilt;
- R²⁴, R²⁵: sind gleich oder verschieden geradkettige oder verzweigte Alkylgruppen mit 1 bis 22, vorzugsweise 1 bis 12 C-Atomen.

Vorzugsweise gilt für die erfindungsgemäßen Polymere:
R¹ = R², R³ = R⁴ = R⁷ = R⁸, R⁵ = R⁶.

Die Herstellung der erfindungsgemäßen Polymere kann dadurch erfolgen, daß man eine Dicarbonylverbindung der Formel (II), 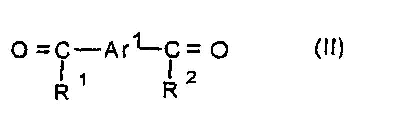 unter Einwirkung eines basischen Kondensationsmittels, d.h. einer Base mit mindestens zwei Äquivalenten einer phosphororganischen Verbindung der Formel (III) umsetzt, 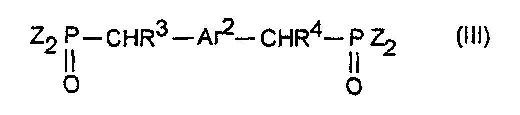 und das dabei überwiegend gebildete Zwischenprodukt der Formel (IV) 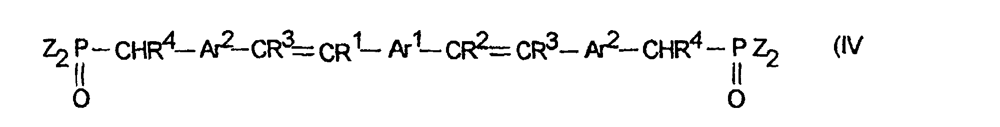 unter Einwirkung einer Base mit einer Dicarbonylverbindung der Formel (V) 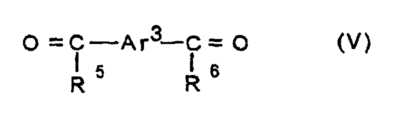 zu einem erfindungsgemäßen Polymer, enthaltend Wiederholeinheiten der Formel (I), polymerisiert, wobei die Symbole dieselben Bedeutungen wie in der Formel (I) haben und Z für Alkoxyreste mit 1 bis 16 C-Atomen, vorzugsweise Ethoxy, oder Arylreste mit 6 bis 10 C-Atomen, vorzugsweise Phenyl, steht.

Dieses Verfahren ist ebenfalls Gegenstand der Erfindung.

Ebenso ist es natürlich möglich und Gegenstand der Erfindung, von einer phosphororganischen Verbindung der Formel (III) auszugehen und diese mit zwei Äquivalenten einen Carbonylverbindung der Formel (II) umzusetzen, was zu einem Zwischenprodukt der Formel (IVa) führt, das entsprechend Carbonylendgruppen trägt und mit einer weiteren phosphororganischen Verbindung der Formel (IIIa) (mit R⁵ und R⁶ statt R³ und R⁴) polymerisiert wird.

Die Kondensationsschritte erfolgen durch Einwirkung einer Base, vorzugsweise einer starken Base, beispielsweise eines Alkoholats, wie Alkalialkoholat, oder Hydrids, wie Natriumhydrid, vorzugsweise von Kalium-tert.-butylat.

Die Polykondensation wird zweckmäßigerweise so vorgenommen, daß man das Gemisch der Ausgangskomponenten in einem Lösungsmittel vorlegt und unter Inertgasatmosphäre und Rühren vorzugsweise mindestens molare Mengen Kondensationsmittel in Lösung oder Suspension einträgt.

Nach einer anderen Arbeitsvariante kann das Kondensationsmittel auch allein oder mit dem Diketon (II) oder (V) in einem Lösungsmittel vorgelegt und die phosphororganische Verbindung zugegeben werden. Als Lösungsmittel werden vorzugsweise Kohlenwasserstoffe, besonders bevorzugt aromatische Kohlenwasserstoffe, wie Benzol, Toluol oder Xylole, verwendet oder polar aprotische Lösungsmittel, bevorzugt Amide, wie N-Methylpyrrolidon (NMP). Die Reaktionstemperatur beträgt vorzugsweise 60 bis 120°C und die Reaktionszeit 5 bis 20 Stunden.

Durch Zusatz von Wasser, gegebenenfalls einer Säure, wie Essigsäure, und Abtrennung der organischen Reaktionsphasen kann die Aufarbeitung erfolgen. Die erhaltenen Kondensationsprodukte können zur Reinigung extrahiert, z.B. mit Alkoholen oder Essigsäure, oder aus einem Lösungsmittel in einem Nichtlösungsmittel gefällt werden.

Allgemein ist dieses Herstellungsverfahren beispielsweise in DD 84 272, Hörhold, H.-H.; Z. Chem. 1972, 12, 41; Hörhold, H.-H.; Bergmann, R.; Gottschaldt, J.; Drefahl, G.: Acta Chim. Acad. Sci. Hung. 81, 239; Hörhold, H.-H.; Bergmann, R.: Advances in the Chemistry of Thermally Stable Polymers, Warszawa, Polish Scientific Publishers 1977, 29-48; Hörhold, H.-H.; Helbig, M.: Makromol. Chem., Macromol. Symp., 1987, 12, 229 und Hörhold, H.-H.; Helbig, M.; Raabe, D.; Opfermann, J.; Scherf, U.; Stockmann, R.; Weiß, D.: Z. Chem. 1987, 27, 126 beschrieben.

Das Zwischenprodukt der Formel (IV) kann nach bekannten, dem Fachmann geläufigen Methoden isoliert werden, ebenso bevorzugt ist es jedoch, die Verbindung der Formel (IV) in situ mit weiterer Dicarbonylverbindung (V) umzusetzen.

Ebenso ist es bevorzugt, Verbindungen der Formel (IV) bzw. (IVa) (mit -COR⁴ als Endgruppen) nach alternativen Synthesemethoden herzustellen, beispielsweise durch Umsetzung einer phosphororganischen Verbindung der Formel (III) mit zwei Äquivalenten einer einseitig, beispielsweise als Acetal oder Ketal, geschützten Dicarbonylverbindung und anschließende Abspaltung der Schutzgruppe. Danach kann weiter, wie oben beschrieben, polymerisiert werden.

Gegenstand der Erfindung sind auch die organische Phosphorverbindung der Formel (IV) bzw. die Dicarbonylverbindung der Formel (IVa), die sich ebenfalls zur Verwendung als Elektrolumineszenzmaterial eignen.

Ebenso Gegenstand der Erfindung sind Umsetzungsprodukte der Verbindung (IV) oder (IVa) mit Monocarbonylverbindungen bzw. monophosphororganischen Verbindungen, wie sie durch die Formel (VI) wiedergegeben werden, 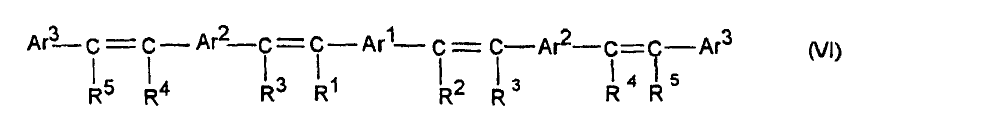 wobei die Symbole die in der Formel (I) angegebenen Bedeutungen haben.

Die Herstellung der Ausgangsverbindungen (II) und (III) erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Die als Kondensationskomponenten benötigten Bis(diphenylphosphinoxide) bzw. Bis(phosphonsäureester) sind beispielsweise unter Anwendung der Michaelis-Arbusov-Reaktion aus den entsprechenden Bis(halogen-methyl)-Verbindungen mit Diphenylphosphinigsäureethylester (C₆H₅)₂P-O-C₂H₅ bzw. mit Triethylphosphit leicht zugänglich.

Neben primären Phosphonaten/Phosphinoxiden sind auch sekundäre geeignet, insbesondere aromatische.

Gegenstand der Erfindung sind daher auch sekundäre Bisphosphorverbindungen der Formel (III), 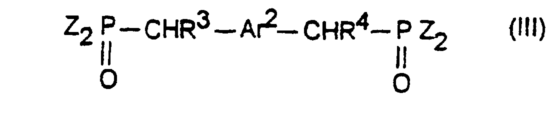 wobei die Symbole folgende Bedeutungen haben:
- Ar²: ist eine- oder mehrkernige, gegebenenfalls substituierte und gegebenenfalls über eine oder mehrere, vorzugsweise eine, Brücke verknüpfte, oder kondensierte Aryl- oder Heteroarylgruppe, vorzugsweise mit 2 bis 100, besonders bevorzugt 2 bis 50, ganz besonders bevorzugt 2 bis 20 Kohlenstoffatomen;
- R³, R⁴, R⁵: sind gleich oder verschieden ein Kohlenwasserstoffrest mit 1 bis 22 Kohlenstoffatomen, der gegebenenfalls substituiert sein kann, vorzugsweise mit F, und auch Heteroatome, vorzugsweise O, enthalten kann;
- Z: ist ein Alkoxyrest mit 1 bis 16 C-Atomen, vorzugsweise Ethoxy, oder ein Arylrest mit 6 bis 10 C-Atomen, vorzugsweise Phenyl.

Bevorzugt ist R³ gleich R⁴.

Weiterhin bevorzugt sind Verbindungen der Formel III, bei denen die Symbole folgende Bedeutungen haben:
- Ar²: ist 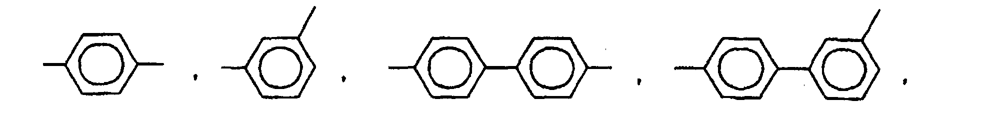 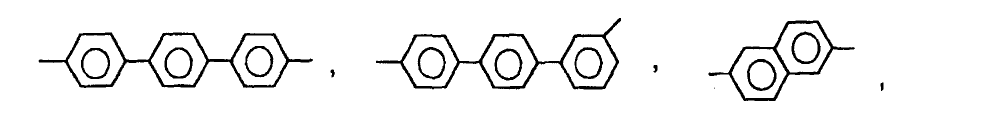 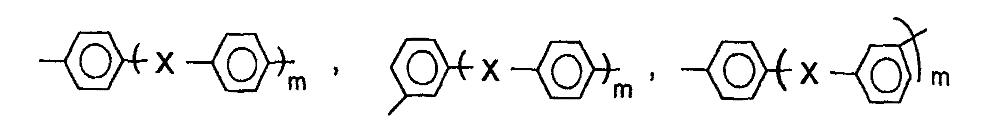 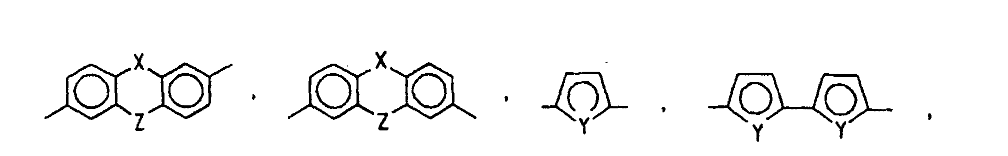 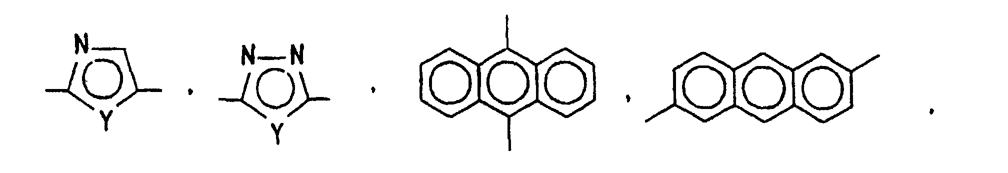 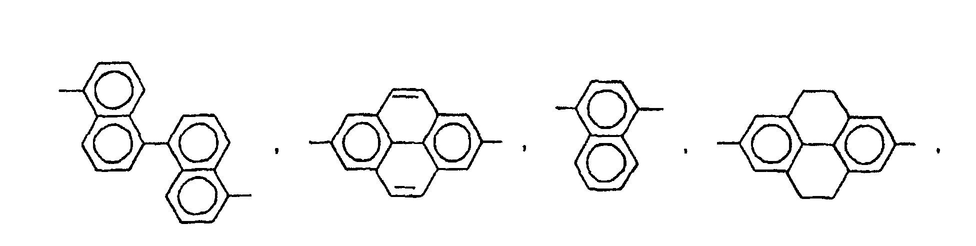 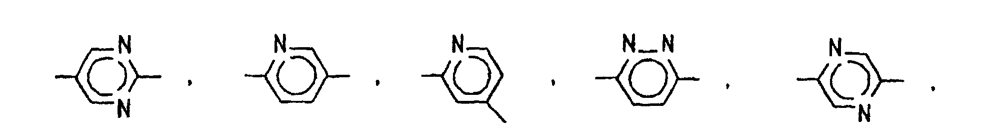 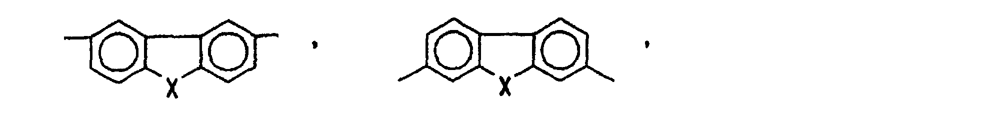
- Ar²: kann dabei gleich oder verschieden mit einem oder mehreren Resten R⁹-R¹⁴ substituiert sein;
- m: ist 1 bis 20, vorzugsweise 1, 2 oder 3, besonders bevorzugt 1;
- X, Z: sind gleich oder verschieden eine Einfachbindung, -O-, -S-, -SO-, -SO₂-, -CO-, -CR⁹R¹⁰-, -CR¹¹=CR¹²-, -CHR¹³, -CHR¹⁴-, -NR^{15-;}
- Y: ist -O-, -S-, oder -NR¹⁵-;
- R⁹-R¹⁴: sind gleich oder verschieden H, -CF₃, -Ph, -O-Ph, -S-Ph, -SO-Ph, -SO₂-Ph, F, Cl, Br, I, -CN oder eine Alkylgruppe mit 1 bis 22, vorzugsweise 1 bis 12, Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können;
- R¹⁵: ist H oder ein Kohlenwasserstoffrest mit 1 bis 22 Kohlenstoffatomen, der gegebenenfalls substituiert sein kann, vorzugsweise mit F, und auch Heteroatome, vorzugsweise O, enthalten kann;

Besonders bevorzugt sind Verbindungen der Formel III, bei denen die Symbole folgende Bedeutungen haben:
- Ar²: ist: 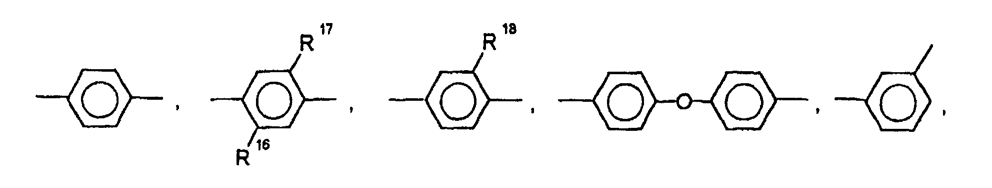 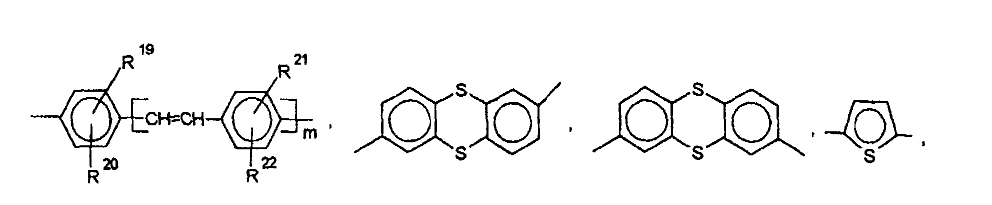 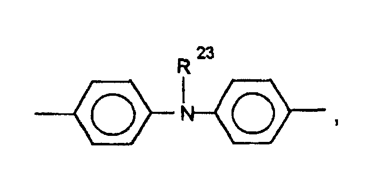 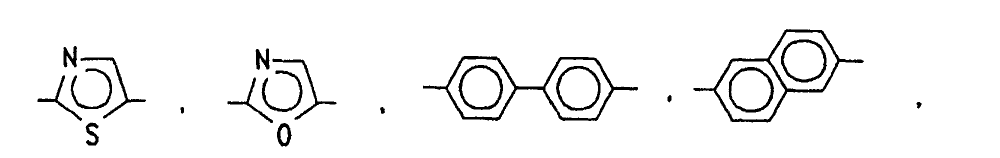
- m: ist 1 bis 20, vorzugsweise 1, 2 oder 3, besonders bevorzugt 1;
- R¹⁶-R²³: sind gleich oder verschieden F, Cl, C₆-C₁₀-Aryl, eine geradkettige oder verzweigte Alkyl- oder Alkoxygruppe mit 1 bis 22, vorzugsweise 1 bis 12, Kohlenstoffatomen, R¹⁹⁻²² können auch H sein.

Ganz besonders bevorzugt ist
- Ar²:: 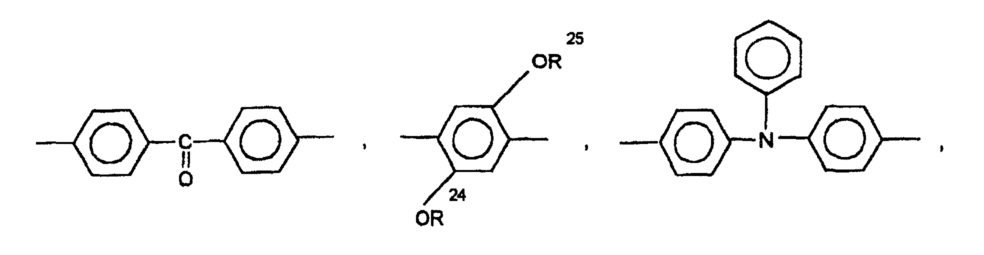 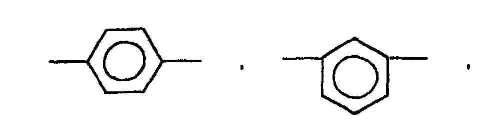
- R²⁴, R²⁵: sind gleich oder verschieden geradkettige oder verzweigte Alkylgruppen mit 1 bis 22, vorzugsweise 1 bis 12 C-Atomen.

Die Synthese von aromatischen Diketonen kann beispielsweise durch die bekannte Friedel-Crafts-Reaktion, mit AlCl₃ als Katalysator, erfolgen, wie in Schema 1 an Hand von drei speziellen Verbindungen beispielhaft gezeigt ist. 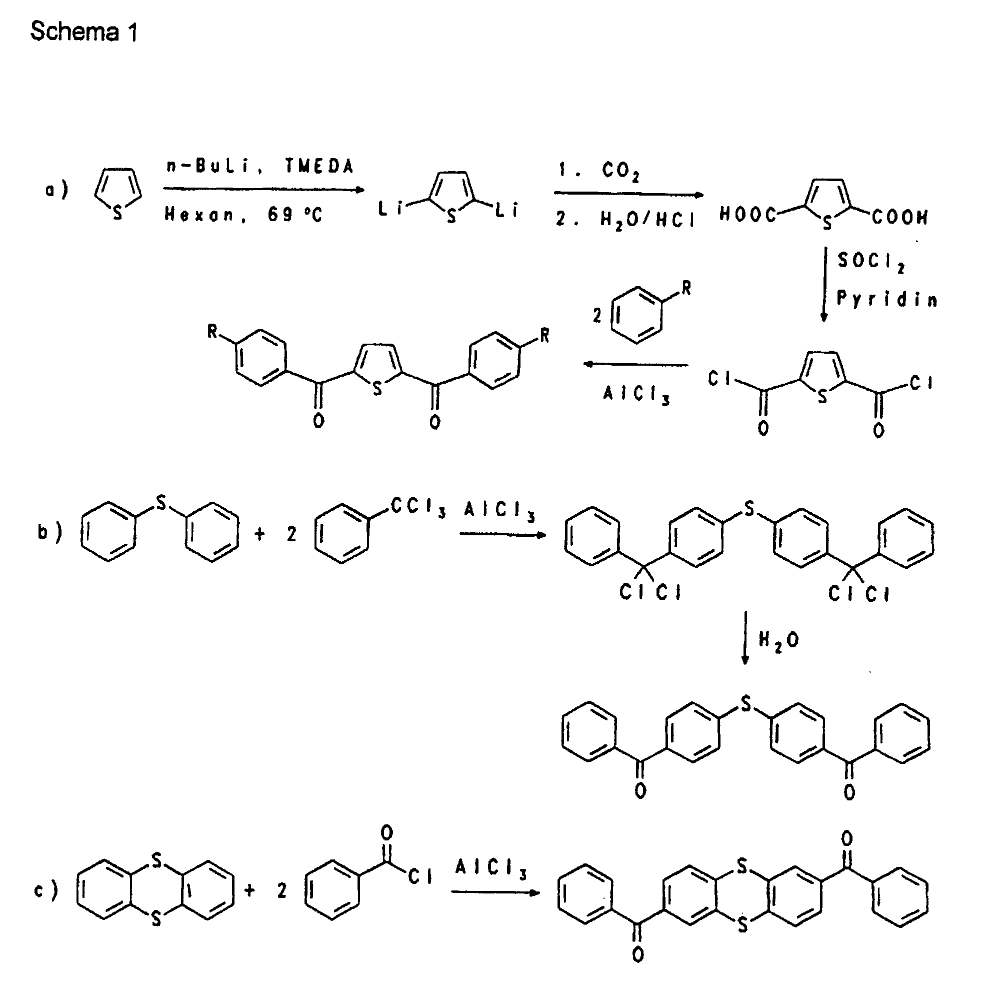 wobei R beispielsweise F oder CF₃ ist.

Eine weitere Variante ist die in Schema 2 beispielhaft dargestellte Grignardreaktion von Arylmagnesiumbromiden mit Dicyanoarylen:

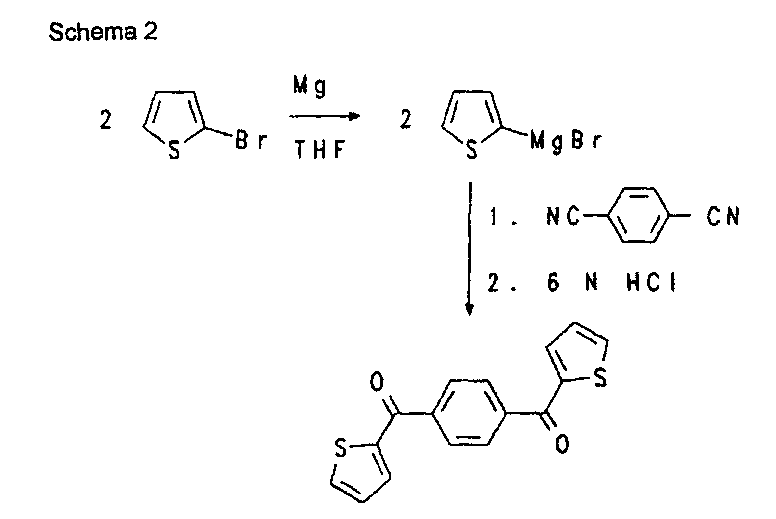

Die Synthese von Bisaldehyden der Formel (II) kann nach verschiedenen, dem Fachmann geläufigen Reaktionstypen erfolgen.

So können Aldehyde beispielsweise aus Bis-carbonsäurederivaten durch kontrollierte Reduktion mit Reduktionsmitteln wie Lithium-tris-alkoxyalanaten oder H₂/Pd ("Rosenmund-Reduktion") erhalten werden: 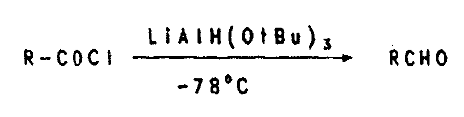 (siehe z.B. Fuson in Patai, "The Chemistry of the Carbonyl Group", Vol. 1, S. 211-232, Interscience, New York 1966) 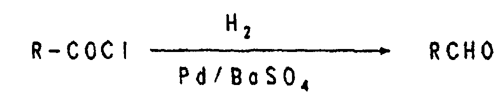 (siehe z.B. Tylander, "Catalytic Hydrogenation over Platinum Metals, S. 398-404, Academic Press, New York 1967).

Aus Bischlormethyl-Vorstufen lassen sich beispielsweise durch die Sommelet-Reaktion Bisaldehyde erhalten 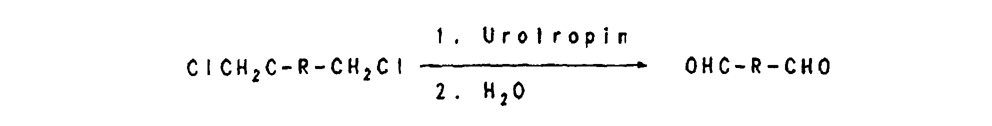 (siehe z.B. Angyal, Organic Reactions 1954, 8, 197), ebenso möglich ist die Swern Oxidation.

Bevorzugt werden jedoch die zugrundeliegende Aromaten einer elektrophilen aromatischen Substitution unterworfen.
Dafür sind zahlreiche Methoden bekannt, beispielsweise die Gattermann-Koch-Reaktion: 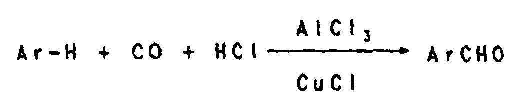 (siehe z.B. Crounse, Organic Reactions 1949, 5, 290-300); die Gattermann Reaktion: 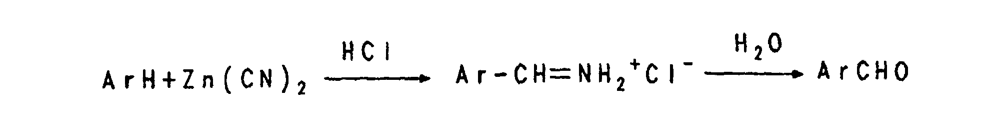 (siehe z.B. Truce, Organ. Reactions 1957, 9, 37-72); oder die Reaktion von Aromaten mit Dichlormethylmethylether: 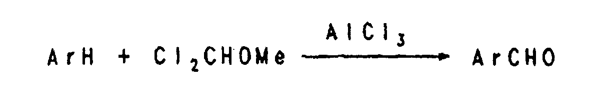 (siehe z.B. Rieche et al., Chem. Ber. 1960, 93, 88 oder Lewin et al., Org. Prep. Proced. Int. 1978, 10, 201).

Bevorzugt ist dabei die Vilsmeier Reaktion, z. B. mit DMF oder N-Methylformanilid: 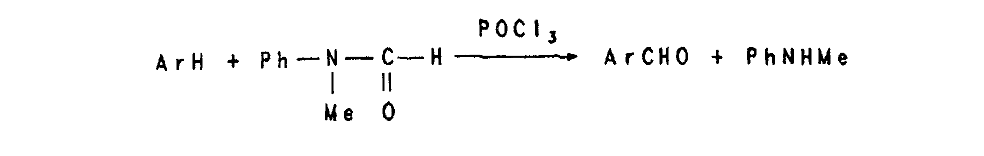 (siehe z.B. Jutz in Advances in Organic Chemistry,Vol. 9, Teil 1 S. 225-342, Böhme, Viche Eds. Interscience, New York 1976, oder Jackson, J. Am. Chem. Soc. 1981, 103, 533).

Polymere, enthaltend Wiederholeinheiten der Formel (I), eignen sich als Elektrolumineszenzmaterialien.

Als Elektrolumineszenzmaterialien im Sinne der Erfindung gelten Materialien, die als aktive Schicht in einer Elektrolumineszenzvorrichtung Verwendung finden können. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch die Verwendung eines Polymers, enthaltend Wiederholeinheiten der Formel (I), als Elektrolumineszenzmaterial.

Um als Elektrolumineszenzmaterialien Verwendung zu finden, werden die Polymere, enthaltend Struktureinheiten der Formel (I), im allgemeinen nach bekannten, dem Fachmann geläufigen Methoden, wie Eintauchen (Dipping) oder Spincoating, in Form eines Films auf ein Substrat aufgebracht.

Ein Gegenstand der Erfindung ist daher auch ein Verfahren zur Herstellung eines Elektrolumineszenzmaterials, dadurch gekennzeichnet, daß man
a) eine Dicarbonylverbindung der Formel (II) 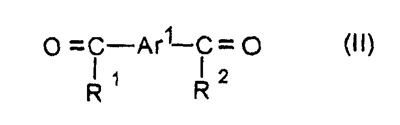 unter Einwirkung eines basischen Kodensationsmittels mit mindestens zwei Äquivalenten einer phosphororganischen Verbindung der Formel (III) umsetzt, 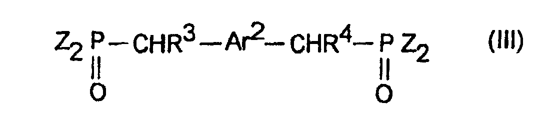
b) das dabei überwiegend gebildete Zwischenprodukt der Formel (IV) 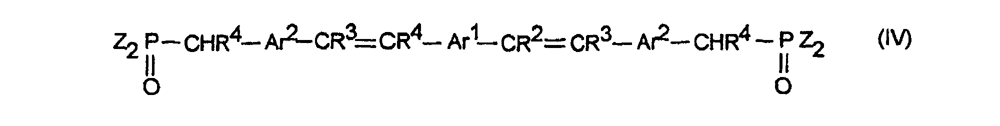 unter Einwirkung einer Base mit einer Dicarbonylverbindung der Formel (V) 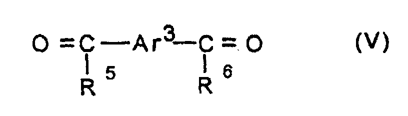 zu einem erfindungsgemäßen Polymer, enthaltend Wiederholeinheiten der Formel (I), polymerisiert, wobei die Symbole dieselben Bedeutungen wie in der Formel (I) haben und Z für Alkoxyreste mit 1 bis 16 C-Atomen, vorzugsweise Ethoxy, oder Arylreste mit 6 bis 10 C-Atomen, vorzugsweise Phenyl, steht, und
c) das so erhaltene Polymer, enthaltend Wiederholeinheiten der Formel (I), in Form eines Films auf ein Substrat, das gegebenenfalls auch weitere Schichten enthält, aufbringt.

Gegenstand der Erfindung ist weiterhin eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO-A 90/13148 oder EP-A 0 443 861 beschrieben.

Sie enthalten üblicherweise eine elektrolumineszierende Schicht zwischen einer Kathode und einer Anode, wobei mindestens eine der Elektroden transparent ist. Zusätzlich können zwischen der elektrolumineszierenden Schicht und der Kathode eine oder mehrere Elektroneninjektions- und/oder Elektronentransportschichten eingebracht sein und/oder zwischen der elektrolumineszierenden Schicht und der Anode eine oder mehrere Lochinjektions- undloder Lochtransportschichten eingebracht sein. Als Kathode können vorzugsweise Metalle oder metallische Legierungen, z.B. Ca, Mg, Al, In, Mg/Ag dienen. Als Anode können Metalle, z.B. Au, oder andere metallisch leitende Stoffe, wie Oxide, z.B. ITO (Indiumoxid/Zinnoxid) auf einem transparentem Substrat, z.B. aus Glas oder einem transparenten Polymer dienen.

Im Betrieb wird die Kathode auf negatives Potential gegenüber der Anode gesetzt. Dabei werden Elektronen von der Kathode in die Elektroneninjektionsschicht/Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu ElektronenlLoch-Paaren, die unter Aussendung von Licht rekombinieren.
Die Farbe des emittierten Lichtes kann durch die als lichtemittierende Schicht verwendeten Materialien variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Die Erfindung wird durch die Beispiele näher erläutert, ohne sie dadurch beschränken zu wollen.

Es bedeuten
- Tg:: Glasübergangstemperatur, gemessen mittels Differential-Scanning-Calorimetry (DSC)
- Mₙ:: Zahlenmittel des Molekulargewichts
- VPO:: Vapor Pressure Osmometry (siehe z.B. Cherdron, Kern, Braun, Praktikum der Makromolekularen Chemie)
- GPC:: Gelpermeationschromatographie, Standard Polystyrol
- MS:: Massenspektrometrie
- UV/VIS:: UV/VIS-Spektroskopie

### Beispiel 1:

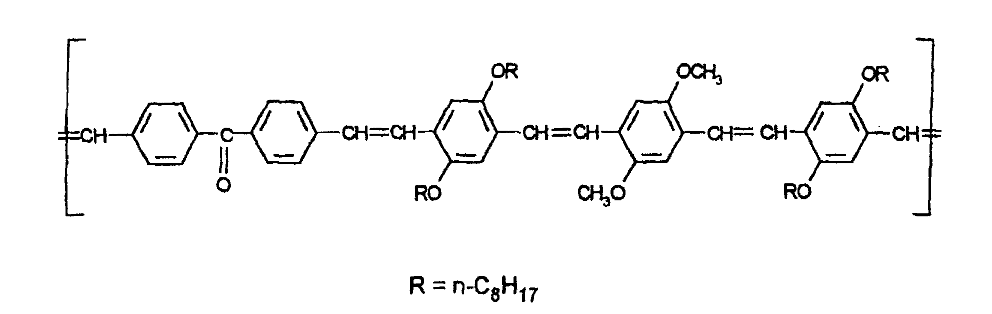

| | |
|---|---|
| 2,5-Dioctoxy-p-xylylen-bis(diethylphosphonat) | 6,53 g (0,010 mol) |
| 2,5-Dimethoxyterephthalaldehyd | 1,00 g (0,005 mol) |
| 4,4'-Diformylbenzophenon | 1,22 g (0,005 mol) |
| Kalium-tert.-butylat | 1,15 g (0,010 mol) (1. Portion) |
| | 1,15 g (0,010 mol) (2. Portion) |

Das Bisphosphonat (0,01 mol) und der Dimethoxyterephthalaldehyd (0,005 mol) werden unter Schutzgas und unter Rühren in 150 ml Toluol auf 80°C erwärmt. Dazu gibt man die erste Portion Kalium-tert.-butylat (0,01 mol), um zunächst die Trimer-Vorstufe herzustellen.
Die Lösung färbt sich rot und fluoresziert orange. Nach zwei Minuten gibt man das Diformylbenzophenon (0,005 mol) in fester Form zu und erhitzt bis zum Siedepunkt. Das restliche Kalium-tert.-butylat (0,01 mol) wird dann in fester Form zugegeben, wobei die Reaktionsmischung stark aufschäumt und viskos wird. Die Farbe der Lösung wird dunkler, die Fluoreszenz intensiver. Man rührt noch eine Stunde in der Siedehitze und gießt dann in 500 ml Methanol ein. Man erhält tief rote Flocken, die abgesaugt werden. Nach dem Trocknen löst man in Chloroform, fällt in Isopropanol aus und erhält ein leuchtend rotes Pulver in 46 %iger Ausbeute.

| | |
|---|---|
| Molmasse (GPC, PS-Standard) | Mn = 4.600 Mw = 14.100 |
| Glastemperatur ( DSC) | Tg = 41°C |
| UV/VIS | λ_{MAX}=452 nm mit breiter Schulter, λ_{0,1MAX}=565 nm, Eg^{OPT}=2,19 eV, Ig ε=4,7 |
| Fluoreszenz | λ_{MAX,EXC}=450 nm, λ_{MAX EM}=535 nm, Eg^{o-o}=2,48 eV, φ_{PL}= 34% |
| Elektrochemie | E^{OX1} = ab 0,70 V vs. Ag/AgCl (reversibel) sehr breit E^{Red1} = 1,24 V vs. Ag/AgCl (reversibel) |
| IR-Spektrum | CH=CH - trans 968 cm-1 |

### Beispiel 2:

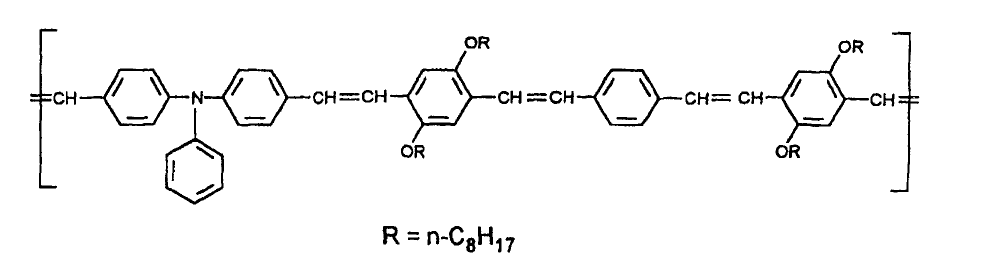

| | |
|---|---|
| 2,5-Dioctoxy-p-xylylen-bis(diethylphosphonat) | 2,00 g (0,0031 mol) |
| Terephthalaldehyd | 0,21 g (0,0015 mol) |
| 4,4'-Diformyltriphenylamin | 0,47 g (0,0015 mol) |
| Kalium-tert.-butylat | 0,35 g (0,0031 mol) = 1. Portion |
| | 0,35 g (0,0031 mol) = 2. Portion |

Das Bisphosphonat (3,1 mmol) und Terephthalaldehyd (1,5 mmol) werden unter Schutzgas und unter Rühren in 150 ml Toluol auf 80°C erwärmt. Dazu gibt man die erste Portion Kalium-tert.-butylat (3,1 mmol), wobei sich zunächst die Trimer-Vorstufe bildet. Die Lösung färbt sich gelblich und fluoresziert hellgrün.

Nach zwei Minuten gibt man den Aminaldehyd (1,5 mmol) in fester Form zu und erhitzt bis zum Siedepunkt. Das restliche Kalium-tert.-butylat (3,1 mmol) wird dann in fester Form zugegeben, wobei die Reaktionsmischung stark aufschäumt und viskos wird. Die Farbe der Lösung wird dunkler, die Fluoreszenz intensiver. Man rührt noch eine Stunde in der Siedehitze, zieht das Lösungsmittel ab, nimmt in Chloroform auf und fällt in Methanol. Man erhält orange Flocken, die abgesaugt und noch vier Stunden mit Methanol extrahiert werden. Nach dem Trocknen erhält man ein leuchtend oranges Pulver in 49 %iger Ausbeute.

| | |
|---|---|
| Molmasse (GPC, PS-Standard) | Mn = 10.500 Mw = 18.700 |
| Glastemperatur (DSC) | Tg = 46°C |
| UV/VIS | λ_{MAX}=452 nm, Ig ε=4,8 |
| Fluoreszenz | λ_{MAX,EXC}=449 nm, λ_{MAX EM}=509 nm, Eg^{o-o}=2,53 eV, φ_{PL} = 66% |
| Elektrochemie | Oxidationspotential E^{OX1} = 0,71 V vs. Ag/AgCl (reversibel durch Cyclovoltammetrie) |
| IR-Spektrum | CH=CH - trans 960 cm⁻¹ |

### Beispiel 3:

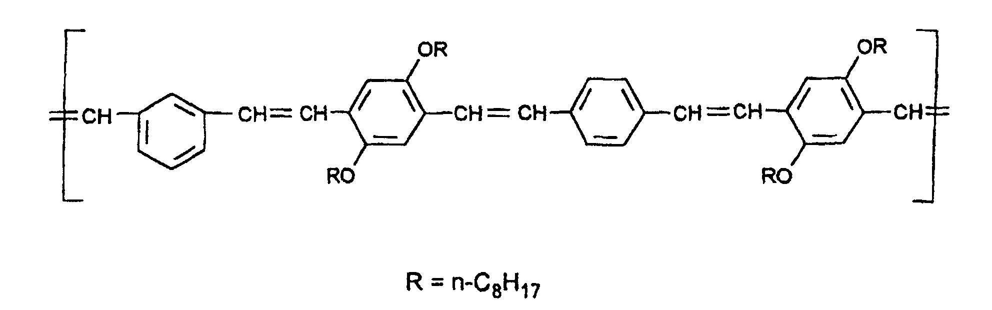

| | |
|---|---|
| 2,5-Dioctoxy-p-xylylen-bis(diethylphosphonat) | 8,00 g |
| Terephthalaldehyd | 0,84 g |
| Isophthalaldehyd | 0,84 g |
| Kalium-tert.-butylat | 1,41 g (1. Portion) |
| | 1,41 g (2. Portion) |

Bisphosphonat und Isophthalaldehyd werden unter Schutzgas und unter Rühren in 150 ml Toluol auf 100°C erwärmt. Dazu gibt man die erste Portion Kalium-tert.-butylat, um zunächst die Trimer-Vorstufe herzustellen. Die Lösung färbt sich gelblich und fluoresziert blau. Nach zwei Minuten gibt man den Terephthalaldehyd in fester Form zu und erhitzt bis zum Siedepunkt. Das restliche Kalium-tert.-butylat wird dann in fester Form zugegeben, wobei die Reaktionsmischung stark aufschäumt und viskos wird. Die Farbe der Lösung wird dunkler, die Fluoreszenz intensiver. Man rührt noch eine Stunde in der Siedehitze und gießt dann in 500 ml Methanol ein. Man erhält orange Flocken, die abgesaugt werden. Nach dem Trocknen nimmt man in Chloroform auf, fällt in Isopropanol aus und extrahiert fünf Stunden mit Methanol. Man erhält ein leuchtend oranges Pulver in 65 %iger Ausbeute (3,8 g).

| | |
|---|---|
| Molmasse (VPO) | Mn = 11.300 |
| UV/VIS | λ_{MAX}=421 nm, Schulter bei 452 nm, Ig ε=4,77 |
| Fluoreszenz | λ_{MAX,EXC}=420 nm, λ_{MAX EM}=507 nm, Schulter bei 540 nm |
| Elektrochemie | E^{OX1} = 0,80 V vs. Ag/AgCl (reversibel), sehr breit |
| IR-Spektrum | CH=CH - trans 964 cm⁻¹ |

### Beispiel 4:

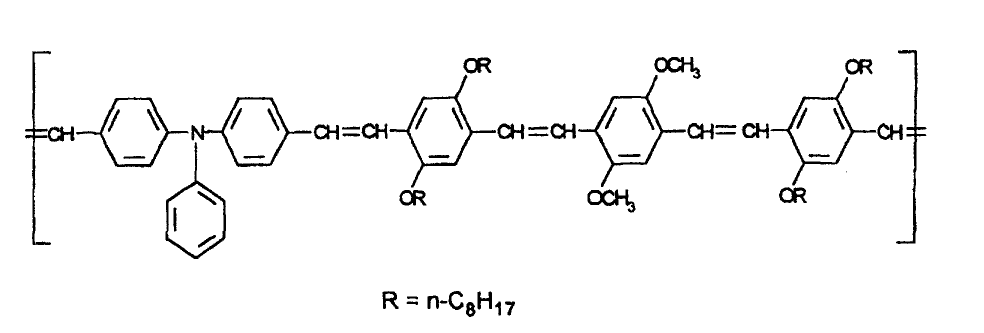

| | |
|---|---|
| 2,5-Dioctoxy-p-xylen-bis(diethylphosphonat) Ro 14 | 4,70 g (0,0074 mol) |
| 2,5-Dimethoxyterephthalaldehyd | 0,72 g (0,0037 mol) |
| 4,4'-Oiformylphenylamin | 1,11 g (0,0037 mol) |
| Kalium-tert.-butylat | 0,83 g (0,0074 mol) = 1. Portion |
| | 0,83 g (0,0074 mol) = 2.Portion |

Das Bisphosphonat (7,4 mmol) und der Dimethoxyterephthalaldehyd (3,7 mmol) werden unter Schutzgas und unter Rühren in 150 ml Toluol auf 80°C erwärmt. Dazu gibt man die erste Portion Kalium-tert.- butylat (7,4 mmol), um zunächst die Trimer-Vorstufe herzustellen. Die Lösung färbt sich orangerot und fluoresziert gelb. Nach zwei Minuten gibt man den Aminaldehyd (3,7 mmol) in fester Form zu und erhitzt bis zum Siedepunkt. Das restliche Kalium-tert.-butylat (7,4 mmol) wird dann in fester Form zugegeben, wobei die Reaktionsmischung stark aufschäumt und viskos wird. Die Farbe der Lösung wird dunkler, die Fluoreszenz intensiver. Man rührt noch eine Stunde in der Siedehitze, zieht das Lösungsmittel ab, nimmt in Chloroform auf und fällt in Methanol ein. Man erhält rote Flocken, die abgesaugt und noch vier Stunden mit Methanol extrahiert werden. Nach dem Trocknen erhält man ein leuchtend rotes Pulver in 45 %iger Ausbeute.

| | |
|---|---|
| Molmasse (GPC, PS-Standard) | Mn = 4.100 Mw = 6.300 |
| UV/VIS | λ_{MAX}=450 nm, λ_{MAX}=537 nm, Eg^{OPT}=2,32, Ig ε=4,8 |
| Fluoreszenz | λ_{MAX,EXC}=440 nm, λ_{MAX EM}=527 nm, Eg^{o-o}=2,54 eV, φ_{PL} = 45% |
| Elektrochemie | E^{OX1} = 0,78 V (reversibel) E^{OX2} = 1,38 V |
| IR-Spektrum | CH=CH - trans bei 965 cm⁻¹ |

### Beispiel 5:

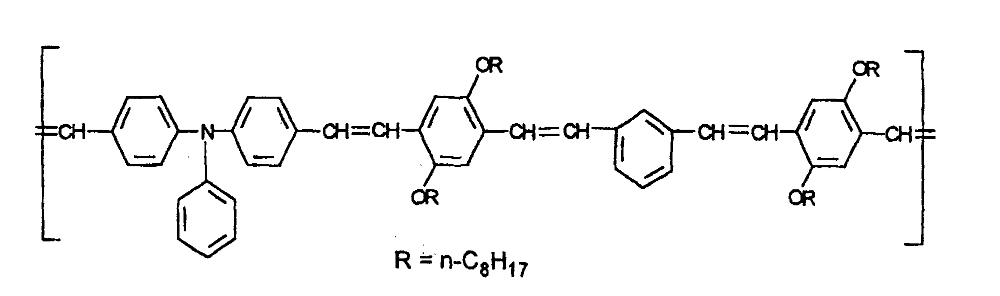

| | |
|---|---|
| 2,5-Dioctoxy-p-xylylen-bis(diethylphosphonat) | 2,00 g (0,0031 mol) |
| Isophthalaldehyd | 0,21 g (0,0015 mol) |
| 4,4'-Diformyltriphenylamin | 0,47 g (0,0015 mol) |
| Kalium-tert.-butylat | 0,35 g (0,0031 mol) (1. Portion) |
| | 0,35 g (0,0031 mol) (2. Portion) |

Das Bisphosphonat und der Isophthalaldehyd werden unter Schutzgas und unter Rühren in 150 ml Toluol auf 80°C erwärmt. Dazu gibt man die erste Portion Kalium-tert.-butylat, um zunächst die Trimer-Vorstufe herzustellen. Die Lösung fäbt sich gelblich und fluoresziert stark blau. Nach zwei Minuten gibt man den Aminaldehyd in fester Form zu und erhitzt bis zum Siedepunkt. Das restliche Kalium-tert.-butylat wird dann in fester Fonn zugegeben, wobei die Reaktionsmischung stark aufschäumt und viskos wird. Die Farbe der Lösung wird dunkler, die Fluoreszenz intensiver. Man rührt noch eine Stunde in der Siedehitze, zieht das Lösungsmittel ab, nimmt in Chloroform auf und fällt in Methanol ein. Man erhält gelbe Flocken, die abgesaugt und noch vier Stunden mit Methanol extrahiert werden. Nach dem Trocknen erhält man ein gelb leuchtendes Pulver in 64 %iger Ausbeute.

| | |
|---|---|
| Molmasse (VPO) | Mn = 7.700 |
| UV/VIS | λ_{MAX}=420 nm (Schulter bei 460 nm) |
| Fluoreszenz | λ_{MAX,EXC} = 417 nm, λ_{MAX,EM} = 493 nm, φ_{Fluo} = 65 % |
| Elektrochemie | E^{OX1} = 0,72 V vs. Ag/AgCl |

### Beispiel 6

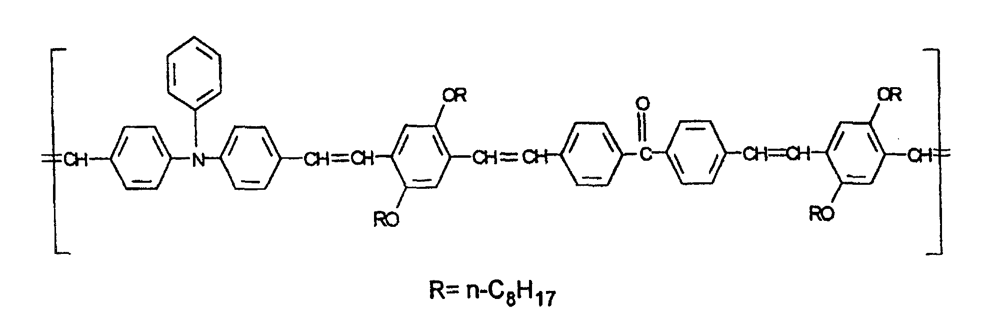

| | |
|---|---|
| 2,5-Dioctoxy-p-xylylen-bis(diethylphosphonat) | 5,32 g (0,0083 mol) |
| 4,4'-Diformylbenzophenon | 1,00 g (0,0041 mol) |
| 4,4'-Diformyltriphenylamin | 1,26 g (0,0041 mol) |
| Kalium-tert.-butylat | 0,94 g (0,0082 mol) (1. Portion) |
| | 0,94 g (0,0083 mol) (2. Portion) |

Das Bisphosphonat (0,0083 mol) und das Diformyltriphenylamin (0,0041 mol) werden unter Schutzgas und unter Rühren in 150 ml Toluol auf 80°C erwärmt. Dazu gibt man die erste Portion Kalium-tert.-butylat (0,0083 mol), um zunächst die Trimer-Vorstufe herzustellen. Die Lösung färbt sich gelblich und fluoresziert grün. Nach zwei Minuten gibt man das Diformylbenzophenon (0,0041 mol) in fester Form zu und erhitzt bis zum Siedepunkt. Das restliche Kalium-tert.-butylat (0,0083 mol) wird dann in fester Form zugegeben, wobei die Reaktionsmischung stark aufschäumt und viskos wird. Die Farbe der Lösung wird dunkler, die Fluoreszenz intensiver. Man rührt noch eine Stunde in der Siedehitze und gießt dann in 500 ml Methanol ein. Man erhält orange Flocken, die abgesaugt werden. Nach dem Trocknen nimmt man in Chloroform auf, fällt in Isopropanol aus und erhält ein leuchtend oranges Pulver in 49 %iger Ausbeute.

| | |
|---|---|
| Molmasse (GPC, PS-Standard) | Mn = 9.200 Mw = 20.600 |
| Glastemperatur ( DSC) | Tg = 57°C |
| UV/VIS | λ_{MAX}=439 nm, λ_{MAX}=495 nm, Eg^{OPT}=2,50 eV, Ig ε=4,95 |
| Fluoreszenz | λ_{MAX,EXC}=449 nm, λ_{MAX EM}=496 nm, Eg^{o-o}=2,57 eV, φ_{PL}= 28 % |
| Elektrochemie | E^{OX1} = 0,73 V vs. Ag/AgCl (reversibel) E^{OX2} = 1,08 V vs. Ag/AgCl (reversibel) E^{Red1} = 1,50 V vs. Ag/AgCl (reversibel) |
| IR-Spektrum | CH=CH - trans 960 cm⁻¹ |

Single layer ITO/Ca 100nm Polymer
U = 16.9 V 153Cd/m² Eff. = 0.038% extern

### Beispiel 7

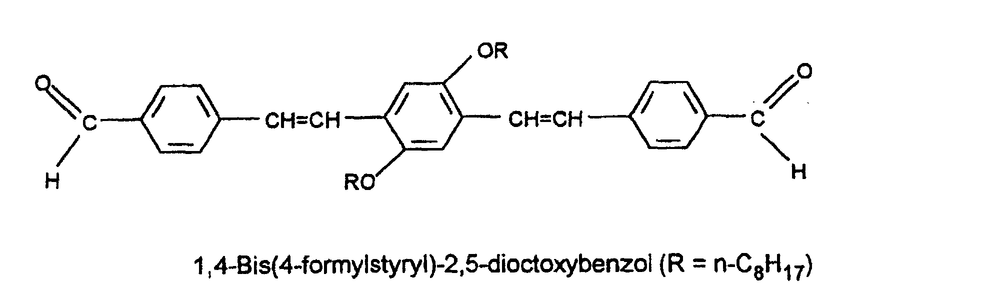

| | |
|---|---|
| 2,5-Dioctoxy-p-xylylen-bis(diethylphosphonat) | 8,8 g (13,8 mmol) |
| 4-Di(ethoxymethyl)benzaldehyd | 5,7 g (27,7 mmol) |
| Kalium-tert.-butylat | 31, g (27,7 mmol) |

8,8 g Bisphosphonat werden zusammen mit 5,7 g des geschützten Aldehyds in 100 ml Toluol aufgelöst und unter Schutzgas auf 80-100°C erwärmt. Dann gibt man das Kalium-tert.-butylat in einer Portion zu, wobei die Reaktionsmischung stark aufschäumt. Die stark fluoreszierende Lösung wird unter Rühren zwei Stunden am Rückfluß erhitzt und danach mit 100 ml 10 %iger Salzsäure hydrolysiert. Die organische Phase wird abgetrennt, mit Wasser gewaschen und danach zur Trockne eingeengt. Der gelb-orange Festkörper wird in heißem Ethanol aufgelöst und mit 20 ml Wasser und einer Spatelspitze p-Toluolsulfonsäure versetzt. Man erhitzt dann noch 20 min am Rückfluß, wobei sich eine klar orange Lösung bildet. Beim vorsichtigen Abkühlen kristallisiert die Verbindung aus (orange Nadeln, Tm=118°C, Ausbeute 76 %).

| | | |
|---|---|---|
| C₄₀H₅₀O₄ (594,76) | ber. C : 80,77 | ber. H :8,47 |
| | gef. C : 80,62 | gef. H : 8,42 |

| | | |
|---|---|---|
| Molmasse durch MS | m/z 594 | |
| Elektrochemie | E^{OX1} = + 1,18 V vs. Ag/AgCl (rev.) E^{Red} = 1,46 V vs. Ag/AgCl (rev.) | |
| UVNIS | λ_{MAX}=420 nm, Ig ε = 4,2 | |
| Fluoreszenz | λ_{MAX}, (Emission) = 485 nm , φ_{PL}= 71 % | |

### Beispiel 8

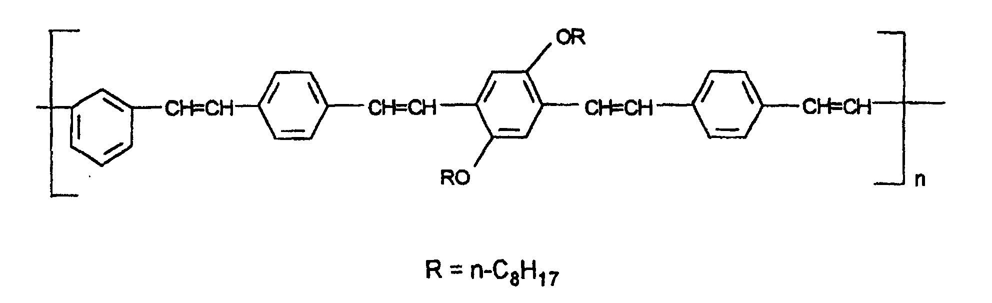

| | |
|---|---|
| 1,4-Bis(4-formylstyryl)-2,5-dioctoxy-benzol | 2,00 g (3,3 mmol) |
| m-Xylylen-bis(diethyl-phosphonat) | 1,27 g (3,3 mmol) |
| Kalium-tert.-butylat | 0,75 g (6,6 mmol) |

2,00 g der Trimer-Vorstufe gemäß Beispiel 7 werden zusammen mit 1,27 g Bisphosphonat unter Schutzgas und Rühren in 50 ml Toluol auf 100°C erwärmt. Dazu gibt man das Kalium-tert.-butylat in einer Portion in fester Form zu, wobei die Mischung stark aufschäumt und viskos wird. Man kocht noch zwei Stunden am Rückfluß und gießt die Mischung dann in 500 ml Methanol ein. Dabei fällt das Polymer im großen gelben Flocken aus. Das Rohprodukt wird dann abgesaugt und anschließend vier Stunden mit Methanol extrahiert (Ausbeute 80 % nach Trocknung im Vakuum).

### Beispiel 9

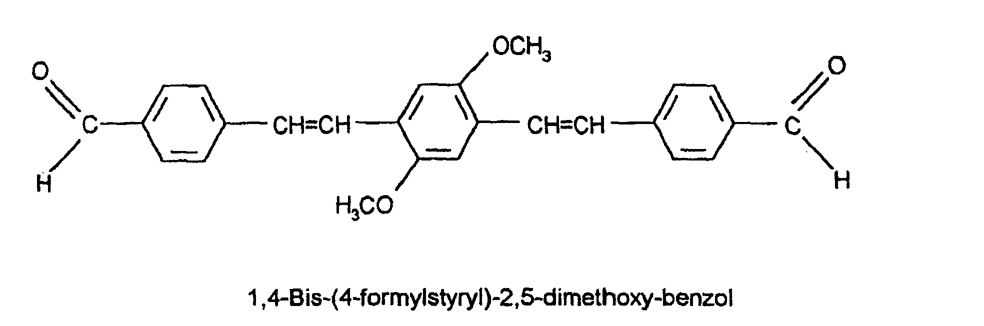

| | |
|---|---|
| 4-(Diethoxymethyl)-benzaldeyd | 8,88 g (42,6 mmol) |
| 2,5-Dimethoxy-p-xylylen-bis(diethylphosphonat) | 9,34 g (21,3 mmol) |
| Kalium-tert-butylat | 5,97 g (53,2 mmol) |

4-(Diethoxymethyl)-benzaldehyd (8,88 g; 42,6 mmol) und das 2,5-Dimethoxy-pxylylen-bis-(diethylphosphonat) (9,34 g; 21,3 mmol) werden in einem trockenen 500 ml Dreihalskolben mit Magnetrührer und Rückflußkühler eingewogen und mit 200 ml Toluol versetzt. Die Apparatur wird mit Argon gespült, und anschließend wird die Reaktionsmischung auf 100 bis 110°C Ölbadtemperatur erhitzt, wobei eine klare Lösung entsteht. Dann wird vorsichtig unter Argon das Kalium-tert.-butylat zugegeben, dabei schäumt die Reaktionsmischung leicht auf. Nach 1/2 Stunde wird der Ansatz so viskos, daß nochmals 50 ml Toluol zugegeben werden. Danach wird 2 Stunden bei obiger Ölbadtemperatur gerührt. Nach Ablauf der Reaktionszeit wird der Ansatz mit verdünnter Essigsäure hydrolysiert. Man trennt die Phasen und schüttelt die Toluolphase mehrmals mit verdünnter Essigsäure aus. Anschließend wäscht man die organische Phase sechsmal mit 100 ml Wasser. Die Toluolphase wird am Wasserabscheider getrocknet. Nach dem Filtrieren wird das Filtrat am Rotationsverdampfer eingeengt, dabei kristallisiert das Produkt aus. Es wird abgesaugt, mit n-Hexan gewaschen und bei 60°C im Vakuum getrocknet. 2,1 g (25 %) oranges Pulver werden erhalten.

| | | |
|---|---|---|
| C₂₆H₂₂O₄ [398,44] | ber. C : 78,37 | H : 5,57 |
| | gef. C: 78,12 | H : 5,86 |

| | |
|---|---|
| λₘₐₓ | 419 nm |
| λ_{max 0,1} | 472 nm |
| IG εₘₐₓ | 4,69 |
| Eₒₚₜ | 2,62 eV |

Fluoreszenzspektrum (in Lösung aufgenommen; Lösungsmittel Dioxan)

| | |
|---|---|
| λₘₐₓ | 420 nm |
| λₐₘ | 484 nm |
| λ₀₋₀ | 460 nm |
| E₀₋₀ | 2,69 eV |
| φ_{PL} | 76 % |

### Beispiel 10

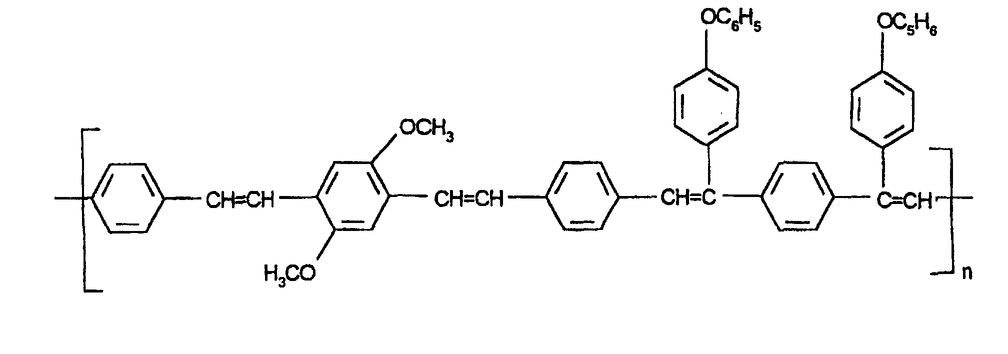

| | |
|---|---|
| 1,4-Bis-(4-formylstyryl)-2,5-dimethoxy-benzol | 1,51 g (3,79 mmol) |
| Benzol-1,4-bis(4-phenoxyphenylmethyldiethylphosphonat) | 2,71 g (3,79 mmol) |
| Kalium-tert.-butylat | 1,28 g (11,4 mmol) |

Bisaldehyd (1,51 g; 3,79 mmol) und Benzol-1,4-bis-(4-phenoxyphenylmethyl)diethylphosphonat) (2,71 g; 3,79 mmol) werden in einem trockenen 250 ml Zweihalskolben mit Magnetrührer und Rückflußkühler eingewogen und mit 70 ml absolutem Toluol versetzt. Die Apparatur wird mit Argon gespült und anschließend wird die Reaktionsmischung auf 130 bis 150°C Ölbadtemperatur erhitzt, wobei eine klare Lösung entsteht. Dann wird vorsichtig unter Argon das Kalium-tert.-butylat zugegeben, dabei schäumt die Reaktionsmischung leicht auf. Nach der Zugabe das Kalium-tert.-butylats wird 3-4 Stunden bei obiger Ölbadtemperatur gerührt. Nach Ablauf der Reaktionszeit wird der Ansatz mit 50 ml 10 %iger Essigsäure hydrolysiert. Man trennt die Phasen und wäscht die organische Phase mehrmals mit Wasser. Anschließend wird die Toluolphase am Wasserabscheider getrocknet. Nach dem Filtrieren wird das Filtrat am Rotationsverdampfer eingeengt und in 500 ml Methanol gefällt. Der gelb-orange Niederschlag wird abgesaugt und mit Methanol und anschließend mit Cyclohexan extrahiert. Nach dem Trocknen im Vakuum bei 100 bis 150°C werden 1,68 g (55 %) Polymer erhalten.

| | |
|---|---|
| Mₙ (GPC) | 8210 g mol⁻¹ |
| M_{w} (GPC) | 29800 g mol⁻¹ |
| M_{w}/Mₙ | 3,63 |
| Tg | > 150°C |

| | |
|---|---|
| λₘₐₓ | 432 nm |
| λ_{max 0,1} | 490 nm |
| IG εₘₐₓ | 4,89 |
| Eₒₚₜ | 2,53 eV |

## Patentansprüche

1. Poly(arylenvinylen)-Terpolymer, enthaltend Wiederholeinheiten der Formel (I), 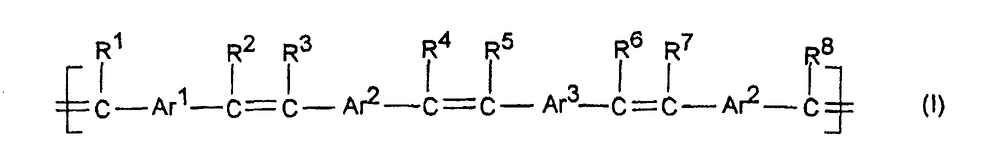 wobei die Symbole folgende Bedeutung haben:
Ar¹, Ar², Ar³ sind gleich oder verschieden ein- oder mehrkemige, gegebenenfalls substituierte und gegebenenfalls über eine oder mehrere Brücken verknüpfte, oder kondensierte Aryl- oder Heteroarylgruppen,
R¹, R², R³, R⁴, R⁵, R6, R⁷, R⁸ sind gleich oder verschieden H oder ein Kohlenwasserstoffrest mit 1 bis 22 Kohlenstoffatomen, der gegebenenfalls substituiert sein kann und auch Heteroatome enthalten kann;
mit der Maßgabe, daß =CR¹-Ar¹-CR²=, =CR³-Ar²-CR⁴= und CR⁵-Ar³-CR⁶= jeweils verschieden voneinander sind.

2. Polymer gemäß Anspruch 1, bestehend aus Wiederholeinheiten der Formel (I).

3. Polymer gemäß Anspruch 1 und/oder 2, **gekennzeichnet durch** 2 bis 1000 Wiederholeinheiten der Formel (I).

4. Polymer gemäß einem oder mehreren der vorhergehenden Ansprüche, wobei die Symbole in der Formel (I) folgende Bedeutungen haben:
Ar¹, Ar², Ar³ sind gleich oder verschieden 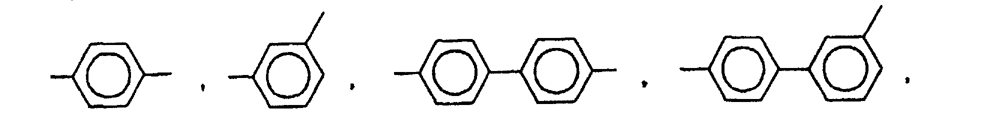 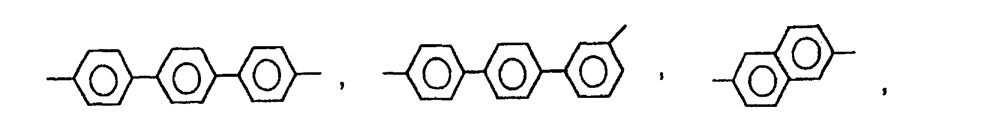 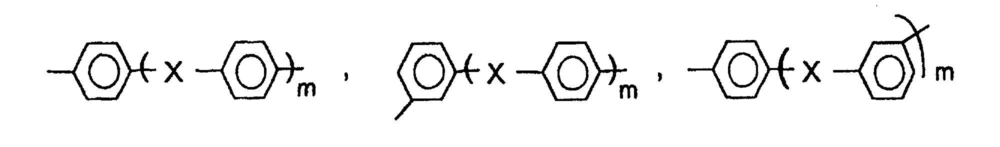 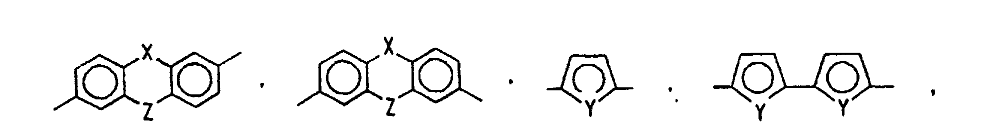 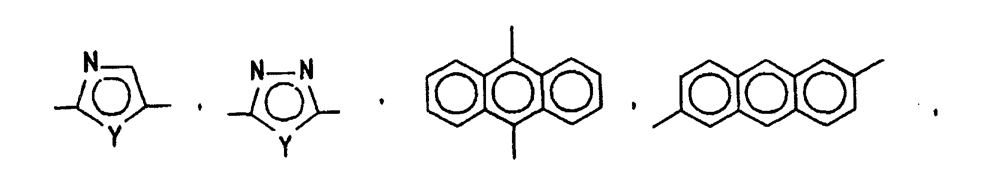 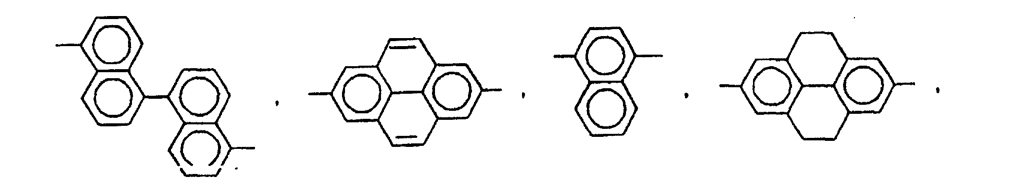 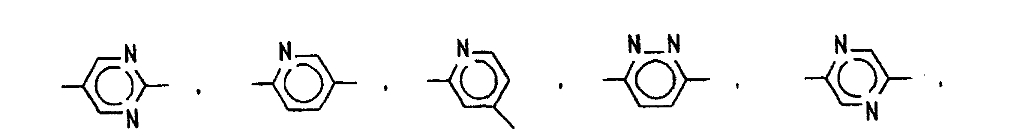 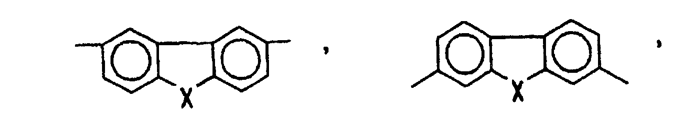
m ist 1 bis 20, vorzugsweise 1, 2 oder 3, besonders bevorzugt 1;
Ar¹, Ar², Ar³ können dabei gleich oder verschieden mit einem oder mehreren Resten R⁹-R¹⁴ substituiert sein;
X, Z sind gleich oder verschieden eine Einfachbindung, -O-, -S-, -SO-, -SO₂-, C=O, -CR⁹R¹⁰-, -CR¹¹=CR¹²-, CHR¹³-CHR¹⁴-, -NR^{15-;}
Y ist -O-, -S-, oder -NR¹⁵-;
R⁹-R¹⁴ sind gleich oder verschieden H, eine Alkylgruppe mit 1 bis 22, vorzugsweise 1 bis 12, Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, -CF₃, -Ph, -O-Ph, -S-Ph, -SO-Ph, -SO₂-Ph, F, Cl, Br, I oder -CN;
R¹⁵ hat, gleich oder verschieden von R¹, die gleichen Bedeutungen wie R¹;
n ist 0, 1 oder 2, vorzugsweise 0 oder 1, besonders bevorzugt 0.

5. Polymer gemäß Anspruch 4, bei dem die Symbole und Indizes in der Formel (I) folgende Bedeutungen haben:
Ar¹, Ar², Ar³ sind verschieden voneinander 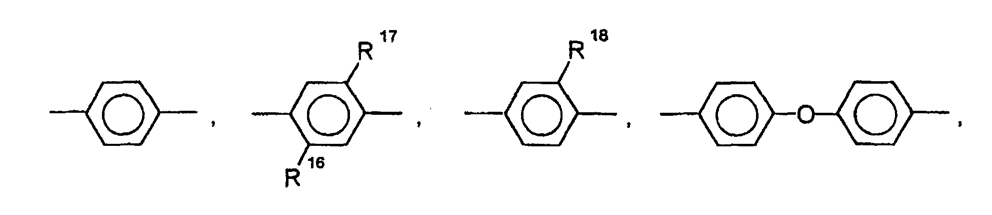 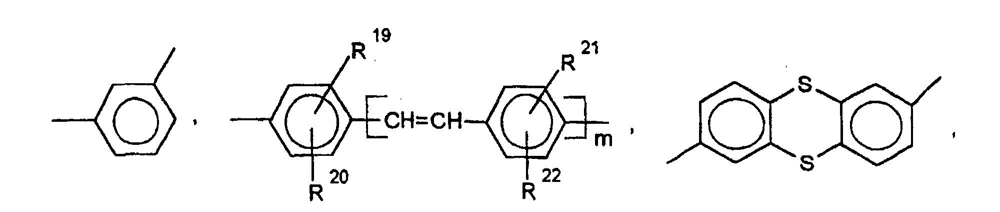 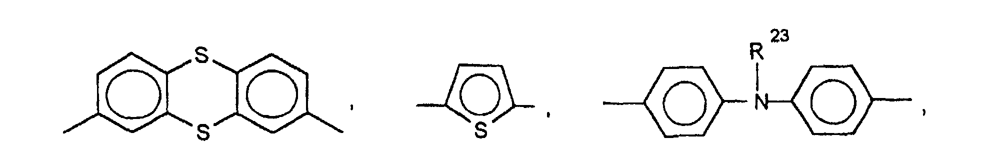 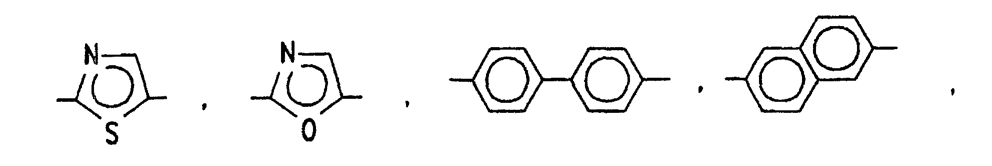
m ist 1 bis 20, vorzugsweise 1, 2 oder 3, besonders bevorzugt 1;
R¹⁶-R²³ sind gleich oder verschieden F, Cl, C₆-C₁₀-Aryl, eine geradkettige oder verzweigte Alkyl- oder Alkoxygruppe mit 1 bis 22, vorzugsweise 1 bis 12, Kohlenstoffatomen, R¹⁹⁻²² können auch H sein.

6. Verfahren zur Herstellung eines Polymers gemäß einem oder mehreren der Ansprüche 1 bis 5, **dadurch gekennzeichnet, daß** man eine Dicarbonylverbindung der Formel (II), 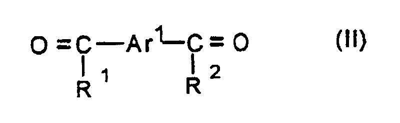 unter Einwirkung einer Base mit mindestens zwei Äquivalenten einer phosphororganischen Verbindung der Formel (III) umsetzt, 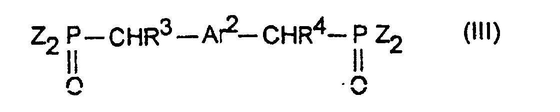 und das dabei gebildete Zwischenprodukt der Formel (IV) 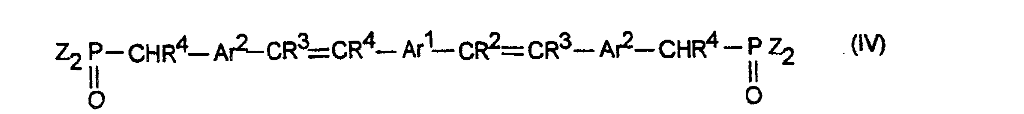 unter Einwirkung einer Base mit einer Dicarbonylverbindung der Formel (V) 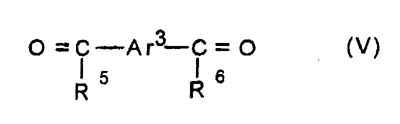 zu einem Polymer, enthaltend Wiederholeinheiten der Formel (I), polymerisiert, wobei die Symbole dieselben Bedeutungen wie in der Formel (I) in Anspruch 1 haben und Z für Alkoxyreste mit 1 bis 16 C-Atomen oder Arylreste mit 6 bis 10 C-Atomen steht.

7. Verwendung eines Polymers gemäß einem oder mehreren der Ansprüche 1 bis 5 als Elektrolumineszenzmaterial.

8. Elektrolumineszenzmaterial, enthaltend ein oder mehrere Polymere gemäß einem oder mehreren der Ansprüche 1 bis 5.

9. Verfahren zur Herstellung eines Elektrolumineszenzmaterials gemäß Anspruch 8, **dadurch gekennzeichnet, daß** man
a) eine Dicarbonylverbindung der Formel (II) 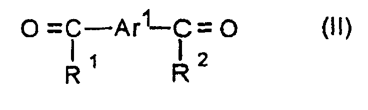 unter Einwirkung einer Base mit mindestens zwei Äquivalenten einer phosphororganischen Verbindung der Formel (III) umsetzt, 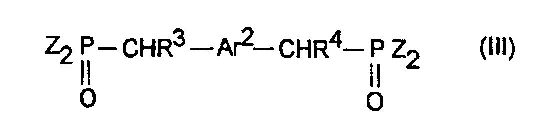
b) das dabei gebildete Zwischenprodukt der Formel (IV) 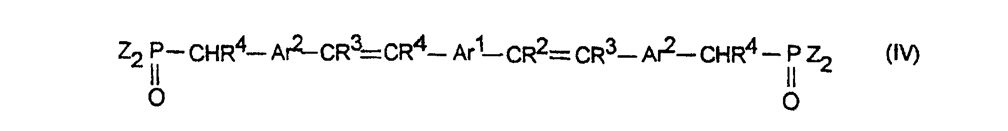 unter Einwirkung einer Base mit einer Dicarbonylverbindung der Formel (V) 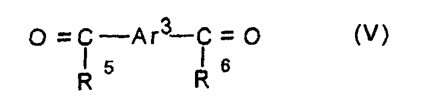 zu einem erfindungsgemäßen Polymer, enthaltend Wiederholeinheiten der Formel (I), polymerisiert, wobei die Symbole dieselben Bedeutungen wie in der Formel (I) in Anspruch 1 haben und Z für Alkoxyreste mit 1 bis 16 C-Atomen oder Arylreste mit 6 bis 10 C-Atomen steht, und
c) das so erhaltene Polymer, enthaltend Wiederholeinheiten der Formel (I), in Form eines Films auf ein Substrat, das gegebenenfalls auch weitere Schichten enthält, aufbringt.

10. Elektrolumineszenzvorrichtung, enthaltend eine oder mehrere aktive Schichten, wobei mindestens eine dieser aktiven Schichten ein oder mehrere Polymere gemäß einem oder mehreren der Ansprüche 1 bis 5 enthält.

11. Sekundäre Bisphosphorverbindungen der Formel (III), 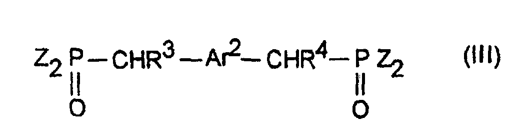 wobei die Symbole folgende Bedeutungen haben:
Ar² ist eine- oder mehrkernige, gegebenenfalls substituierte und gegebenenfalls über eine oder mehrere, vorzugsweise eine, Brücke verknüpfte, oder kondensierte Aryl- oder Heteroarylgruppe;
R³, R⁴, R⁵ sind gleich oder verschieden ein Kohlenwasserstoffrest mit 1 bis 22 Kohlenstoffatomen, der gegebenenfalls substituiert sein kann und auch Heteroatome enthalten kann;
Z ist ein Alkoxyrest mit 1 bis 16 C-Atomen oder ein Arylrest mit 6 bis 10 C-Atomen.

## Claims

1. A poly(arylene-vinylene) terpolymer comprising repeating units of the formula (I), 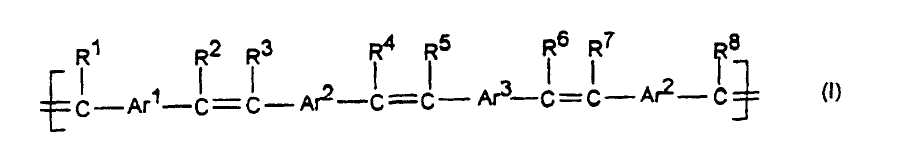 where the symbols have the following meanings:
Ar¹, Ar², Ar³ are identical or different and are monocyclic or polycyclic, substituted or unsubstituted aryl or heteroaryl groups which may be linked via one or more bridges or be fused,
R¹, R², R³, R⁴, R⁵, R⁶, R⁷, R⁸ are identical or different and are each H or a hydrocarbon radical having from 1 to 22 carbon atoms which may be substituted and may also contain heteroatoms;
with the proviso that =CR¹-Ar¹-CR²=, =CR³-Ar²CR⁴= and CR⁵-Ar³-CR⁶= are each different from one another.

2. A polymer as claimed in claim 1 consisting of repeating units of the formula (I).

3. A polymer as claimed in claim 1 and/or 2 having from 2 to 1000 repeating units of the formula (I).

4. A polymer as claimed in one or more of the preceding claims, wherein the symbols in the formula (I) have the following meanings:
Ar¹, Ar², Ar³ are identical or different and are 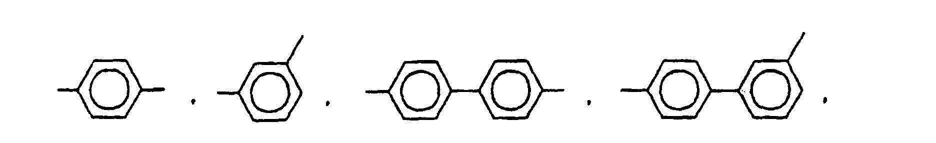 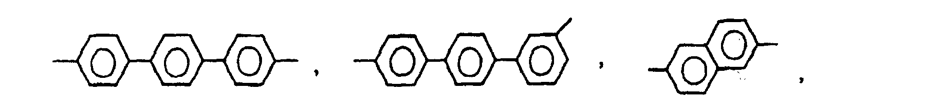 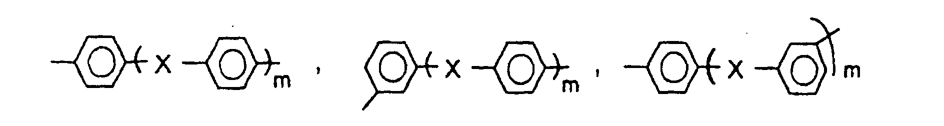 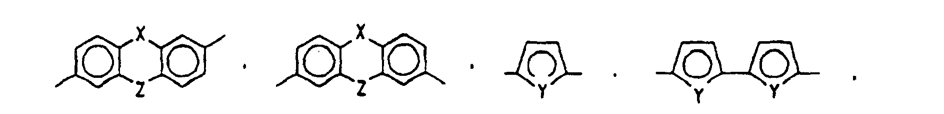 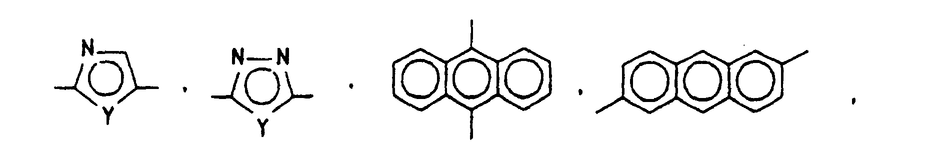 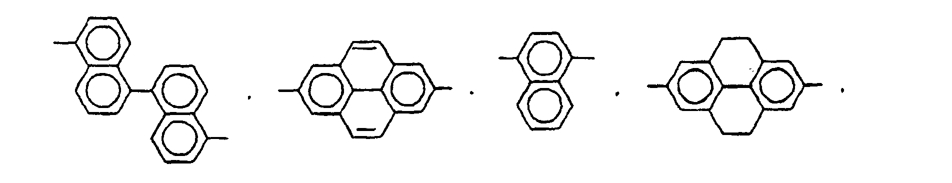 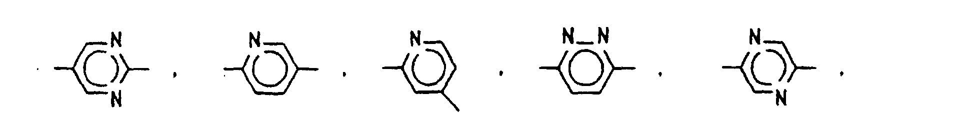 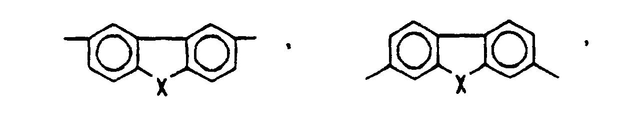
m is from 1 to 20, preferably 1, 2 or 3, particularly preferably 1;
Ar¹, Ar², Ar³ may be substituted by one or more identical or different radicals R⁹-R¹⁴;
X, Z are identical or different and are each a single bond, -O-, -S-, -SO-, -SO₂-, -CO-, -CR⁹R¹⁰-, -CR¹¹=CR¹²-, -CHR¹³-, -CHR¹⁴-, -NR¹⁵-;
Y is -O-, -S-, or -NR¹⁵-;
R⁹-R¹⁴ are identical or different and are each H, an alkyl group having from 1 to 22, preferably from 1 to 12, carbon atoms, where one or two nonadjacent CH₂ groups may also be replaced by -O-, -S-, -CO-, -CO-O-, -O-OC- or -Si(CH₃)₂- or -CF₃, -Ph, -O-Ph, -S-Ph, -SO-Ph, -SO₂-Ph, F, Cl, Br, I or -CN;
R¹⁵ is as defined for R¹ and may be identical to or different from R¹;
n is 0, 1 or 2, preferably 0 or 1, particularly preferably 0.

5. A polymer as claimed in claim 4 in which the symbols and indices in the formula (I) have the following meanings:
Ar¹, Ar², Ar³ are identical or different and are 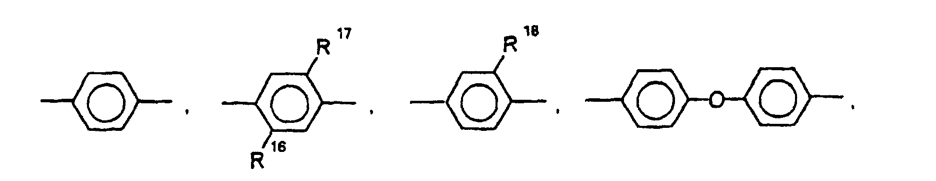 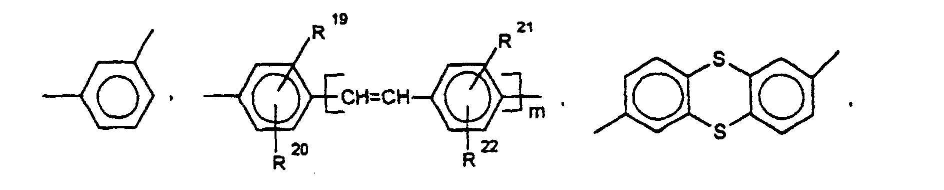 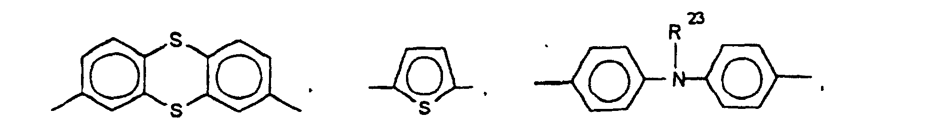 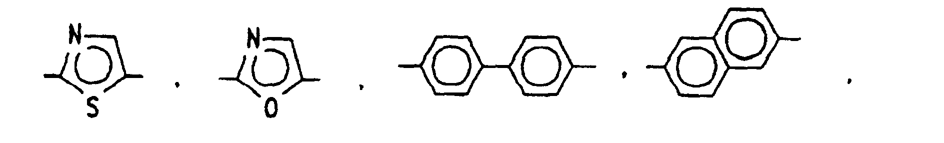
m is from 1 to 20, preferably 1, 2 or 3, particularly preferably 1;
R¹⁶-R²³ are identical or different and are each F, Cl, C₆-C₁₀-aryl, a straight-chain or branched alkyl or alkoxy group having from 1 to 22, preferably from 1 to 12, carbon atoms and R¹⁹⁻²² can also be H.

6. A process for preparing a polymer as claimed in one or more of claims 1 to 5, which comprises reacting a dicarbonyl compound of the formula (II), 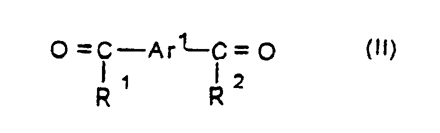 with at least two equivalents of an organophosphorus compound of the formula (III), 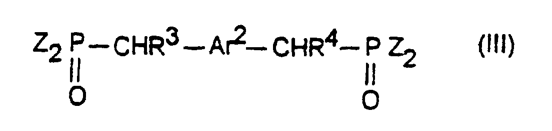 in the presence of a base, and polymerizing the resulting intermediate of the formula (IV) 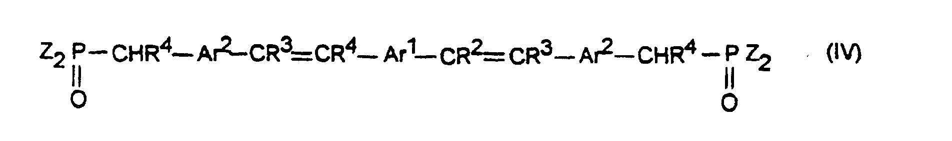 with a dicarbonyl compound of the formula (V) 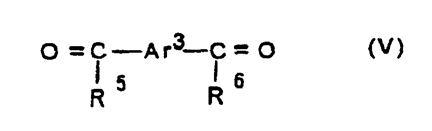 in the presence of a base to give a polymer comprising repeating units of the formula (I), where the symbols have the same meanings as in the formula (I) in claim 1 and Z are alkoxy radicals having from 1 to 16 carbon atoms or aryl radicals having from 6 to 10 carbon atoms.

7. The use of a polymer as claimed in one or more of claims 1 to 5 as electroluminescence material.

8. An electroluminescence material comprising one or more polymers as claimed in one or more of claims 1 to 5.

9. A process for producing an electroluminescence material as claimed in claim 8, which comprises
a) reacting a dicarbonyl compound of the formula (II) 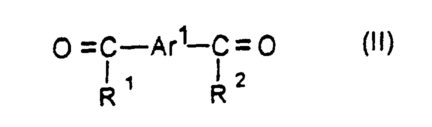 with at least two equivalents of an organophosphorus compound of the formula (III) 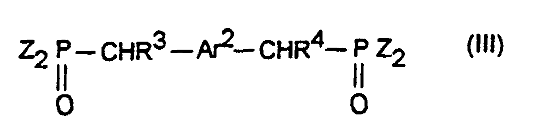 in the presence of a base,
b) polymerizing the resulting intermediate of the formula (IV) 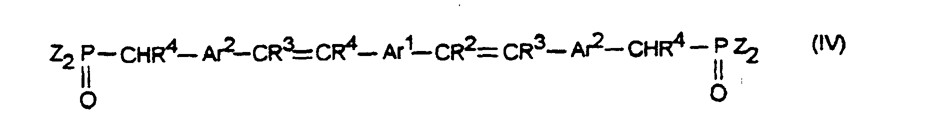 with a dicarbonyl compound of the formula (V) 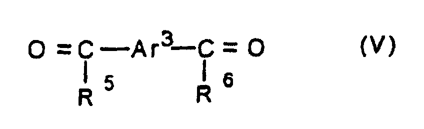 in the presence of a base to give a polymer comprising repeating units of the formula (I), where the symbols have the same meanings as in the formula (I) in claim 1 and Z are alkoxy radicals having from 1 to 16 carbon atoms or aryl radicals having from 6 to 10 carbon atoms and
c) applying the resulting polymer comprising repeating units of the formula (I) in the form of a film to a substrate which, if desired, also comprises further layers.

10. An electroluminescence device comprising one or more active layers, wherein at least one of these active layers comprises one or more polymers as claimed in one or more of claims 1 to 5.

11. A secondary bisphosphorus compound of the formula (III), 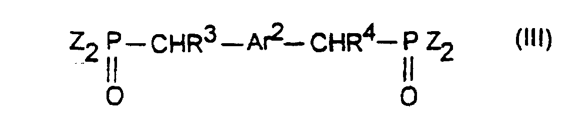 where the symbols have the following meanings:
Ar² is a monocyclic or polycyclic, substituted or unsubstituted aryl or heteroaryl group which may be linked via one or more, preferably one, bridge or fused;
R³, R⁴, R⁵ are identical or different and are each a hydrocarbon radical having from 1 to 22 carbon atoms, which may be unsubstituted or substituted and may also contain heteroatoms;
Z is an alkoxy radical having from 1 to 16 carbon atoms or an aryl radical having from 6 to 10 carbon atoms.

## Revendications

1. Terpolymère poly(aylènevinylène), contenant des motifs de répétition de formule (I), 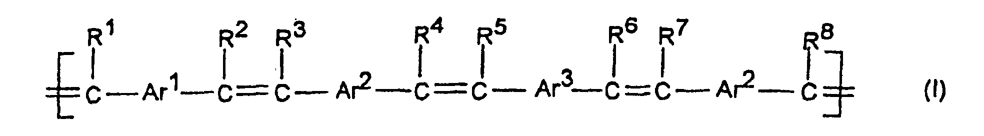 dans laquelle les symboles ont la signification suivante :
Ar¹, Ar², Ar³ sont, identiques ou différents, des groupes aryle ou hétéroaryle, mono- ou polycycliques, éventuellement substitués et éventuellement reliés par un ou plusieurs ponts, ou condensés,
R¹, R², R³, R⁴, R⁵, R⁶, R⁷, R⁸ sont, identiques ou différents, H ou un groupe hydrocarboné ayant de 1 à 22 atomes de carbone, qui peut être éventuellement substitué et aussi contenir des hétéroatomes ;
avec la condition que =CR¹-Ar¹-CR²=, =CR³-Ar²-CR⁴= et =CR⁵-Ar³-CR⁶= soient respectivement différents les uns des autres.

2. Polymère selon la revendication 1, constitué de motifs de répétition de formule (I).

3. Polymère selon la revendication 1 et/ou 2, **caractérisé par** 2 à 1000 motifs de répétition de formule (I).

4. Polymère selon une ou plusieurs des revendications précédentes, dans lequel les symboles dans la formule (I) ont les significations suivantes : Ar¹, Ar², Ar³ sont, identiques ou différents, 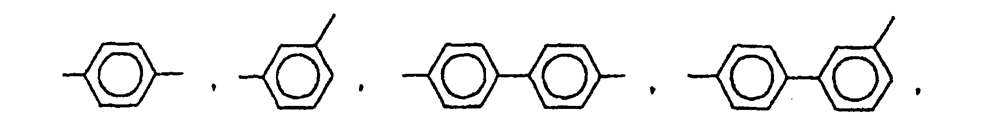 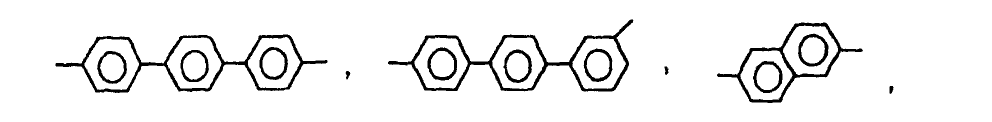 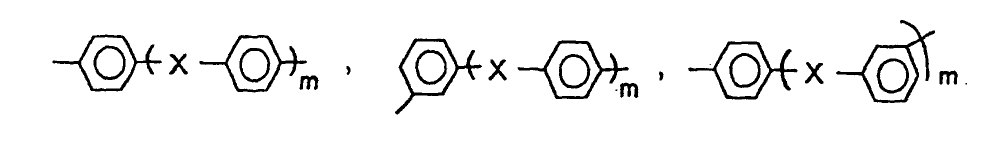 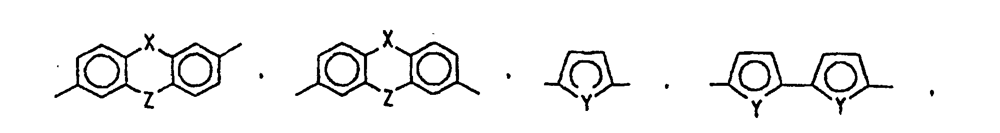 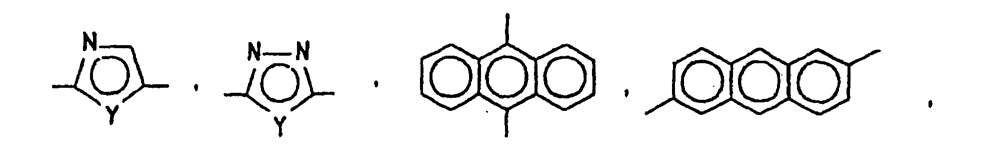 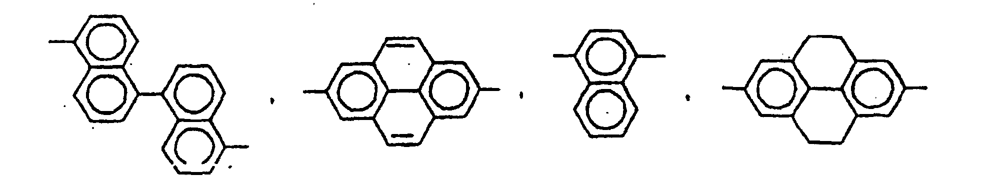 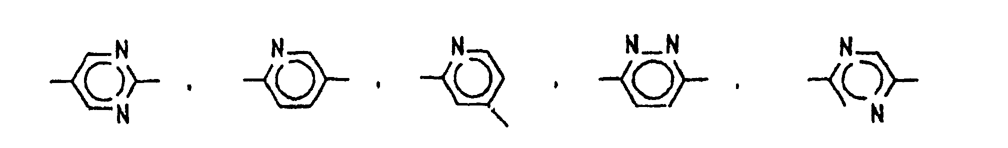 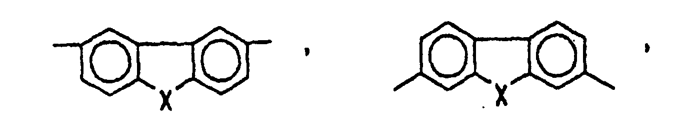
m est 1 à 20, de préférence 1, 2 ou 3, de manière particulièrement préférée 1 ;
Ar¹, Ar², Ar³ peuvent alors, identiques ou différents, être substitués par un ou plusieurs groupes R⁹-R¹⁴ ;
X, Z sont, identiques ou différents, une liaison simple, -O-, -S-, -SO-, -SO₂-, C=O, -CR⁹R¹⁰-, -CR¹¹=CR¹²-, -CHR¹³-CHR¹⁴-, -NR¹⁵- ;
Y est -O-, -S- ou -NR¹⁵- ;
R⁹-R¹⁴ sont, identiques ou différents, H, un groupe alkyle ayant de 1 à 22, de préférence de 1 à 12, atomes de carbone, dans lesquels aussi un ou deux groupes CH₂ non voisins peuvent être remplacés par -O-, -S-, -CO-, -CO-O-, -O-OC ou -Si(CH₃)₂-, -CF₃, -Ph, -O-Ph, -S-Ph, -SO-Ph, -SO₂-Ph, F, Cl, Br, I ou -CN ;
R¹⁵ a, identique ou différent de R¹, les mêmes significations que R¹ ;
n est 0, 1 ou 2, de préférence 0 ou 1, de manière particulièrement préférée 0.

5. Polymère selon la revendication 4, dans lequel les symboles et indices dans la formule (I) ont les significations suivantes :
Ar¹, Ar², Ar³ sont, identiques ou différents, 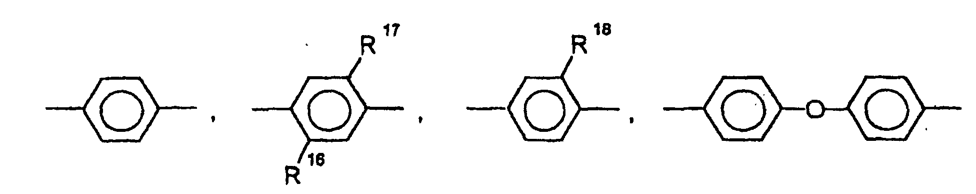 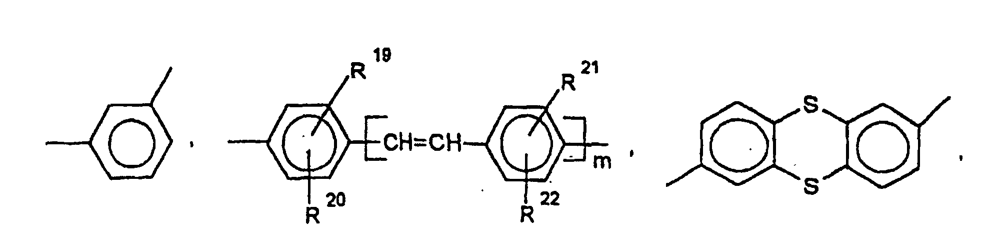 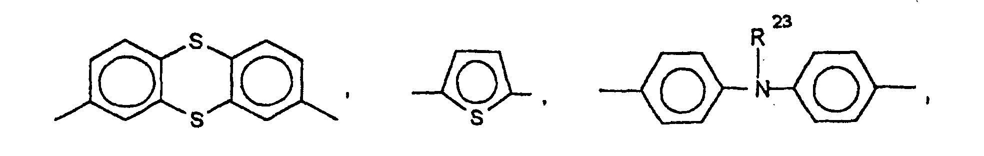 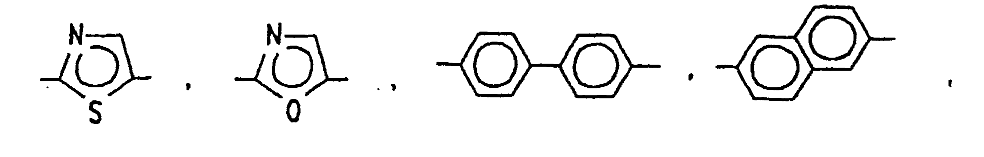
m est 1 à 20, de préférence 1, 2 ou 3, de manière particulièrement préférée 1 ;
R¹⁶-R²³ sont, identiques ou différents, F, Cl, un groupe aryle en C₆-C₁₀, un groupe alkyle ou alcoxy à chaîne linéaire ou ramifiée ayant de 1 à 22, de préférence de 1 à 12, atomes de carbone, R¹⁹⁻²² peuvent aussi être H.

6. Procédé pour la préparation d'un polymère selon une ou plusieurs des revendications 1 à 5, **caractérisé en ce qu'**on met à réagir un composé dicarbonyle de formule (II) 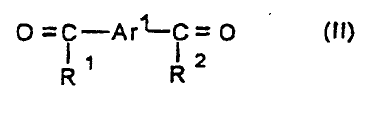 sous l'action d'une base avec au moins deux équivalents d'un composé organique phosphoré de formule (III), 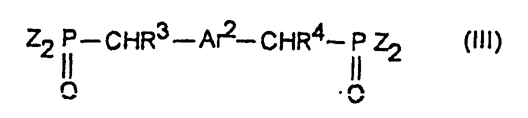 et **en ce qu'**on polymérise le produit intermédiaire de formule (IV) alors formé 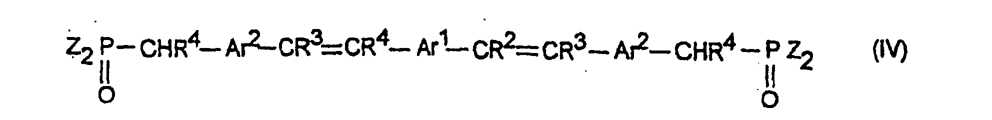 sous l'action d'une base avec un composé dicarbonyle de formule (V) 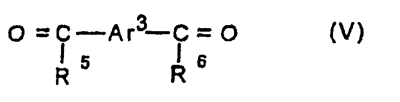 pour obtenir un polymère, contenant des motifs de répétition de formule (I), dans lequel les symboles ont les mêmes significations qu'à la formule (I) dans la revendication 1 et Z représente des groupes alcoxy ayant de 1 à 16 atomes de carbone ou des groupes aryle ayant de 6 à 10 atomes de carbone.

7. Utilisation d'un polymère selon une ou plusieurs des revendications 1 à 5 comme matériau électroluminescent.

8. Matériau électroluminescent, contenant un ou plusieurs polymères selon une ou plusieurs des revendications 1 à 5.

9. Procédé pour la préparation d'un matériau électroluminescent selon la revendication 8, **caractérisé en ce qu'**on met à réagir
a) un composé dicarbonyle de formule (II) 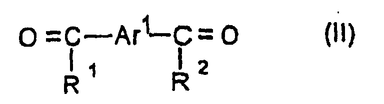 sous l'action d'une base avec au moins deux équivalents d'un composé organique phosphoré de formule (III) 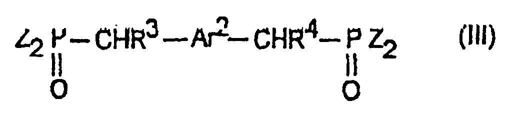
b) on polymérise le produit intermédiaire de formule (IV) alors formé 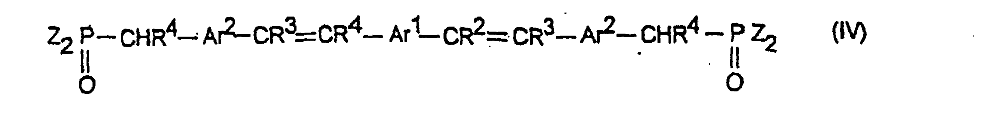 sous l'action d'une base avec un composé dicarbonyle de formule (V) 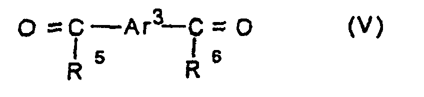 pour obtenir un polymère selon l'invention, contenant des motifs de répétition de formule (I), dans lequel les symboles ont les mêmes significations que dans la formule (I) de la revendication 1 et Z représente des groupes alcoxy ayant de 1 à 16 atomes de carbone ou des groupes aryle ayant de 6 à 10 atomes de carbone, et
c) on dépose le polymère ainsi obtenu, contenant des motifs de répétition de formule (I), sous la forme d'un film sur un substrat, qui contient éventuellement encore d'autres couches.

10. Dispositif électroluminescent, contenant une ou plusieurs couches actives, dans lequel au moins une de ces couches actives contient un ou plusieurs polymères selon une ou plusieurs des revendications 1 à 5.

11. Composés biphosphorés secondaires de formule (III), 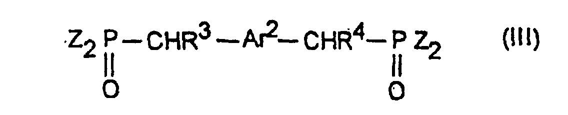 dans laquelle les symboles ont les significations suivantes :
Ar² représente un groupe aryle ou hétéroaryle, mono- ou polycyclique, éventuellement substitué et éventuellement couplé via un ou plusieurs ponts, de préférence un, ou condensé ;
R³, R⁴, R⁵ sont, identiques ou différents, un groupe hydrocarboné ayant de 1 à 22 atomes de carbone, qui peut être éventuellement substitué et encore peut contenir des hétéroatomes ;
Z est un groupe alcoxy ayant de 1 à 16 atomes de carbone ou un groupe aryle ayant de 6 à 10 atomes de carbone.
